# EUROPEAN PATENT APPLICATION

(11) **EP 4 488 284 A1**
(43) Date of publication of application: **08.01.2025**
(21) Application number: 23763292.2
(22) Date of filing: 20.02.2023
(51) Int. Cl.: C07K 7/08, A61K 35/76, A61K 38/08, A61K 38/10, A61K 45/00, A61K 47/64, A61K 48/00, A61P 21/00, A61P 25/28, A61P 35/00, C07K 7/06, C12N 15/11, C12N 15/85, C12N 15/86

(54) **PRORENIN RECEPTOR PEPTIDE, CONJUGATE, AND PHARMACEUTICAL COMPOSITION**

(30) Priority: 03.03.2022 JP 2022032709
(71) Applicant: National University Corporation Kagawa University, Takamatsu-shi, Kagawa 760-8521 (JP)
(72) Inventor: NISHIYAMA Akira, Kita-gun, Kagawa 761-0793 (JP); KITADA Kento, Kita-gun, Kagawa 761-0793 (JP); RAHMAN Asadur, Kita-gun, Kagawa 761-0793 (JP); NAKAGAMI Hironori, Suita-shi, Osaka 565-0871 (JP); HAYASHI Hiroki, Suita-shi, Osaka 565-0871 (JP)
(74) Representative: J A Kemp LLP
(86) International application number: PCT/JP2023/005966
(87) International publication number: WO 2023/167033

(57) **Abstract**

Provided is a prorenin receptor peptide with enhanced inducibility of antibodies against prorenin receptors. The prorenin receptor peptide of the present disclosure includes a polypeptide of following (P1), (P2), or (P3): (P1) a polypeptide consisting of an amino acid sequence of any one of SEQ ID NO: 1-3; (P2) a polypeptide consisting of an amino acid sequence of any one of SEQ ID NO: 1-3 in which one or two or three amino acids are deleted, substituted, or added, or (P3) a polynucleotide consisting of an amino acid sequence having 80% or more of identity to an amino acid sequence of any one of SEQ ID NO: 1-3.

## Description

### TECHNICAL FIELD

The present invention relates to a prorenin receptor peptide, a conjugate, and a pharmaceutical composition.

### BACKGROUND ART

Abnormality in Wnt/β-catenin pathway may cause a variety of diseases. The abnormality in the Wnt/β-catenin pathway may result in, for example, tumors (benign tumours) such as familial adenomatous polyposis, osteoporosis, or the like (Non Patent Literatures 1 to 4). In recent years, it has been attempted to cutoff the Wnt/β-catenin to treat diseases resulting from abnormality in the Wnt/β-catenin pathway.

### Citation List

### Non Patent Literature

Non Patent Literature 1: Caldwell, G. M., et al. "Wnt signalling in adenomas of familial adenomatous polyposis patients." British journal of cancer 103.6 (2010): 910-917.
Non Patent Literature 2: Obrador-Hevia, Antonia, et al. "Oncogenic KRAS is not necessary for Wnt signalling activation in APC-associated FAP adenomas." The Journal of pathology 221.1 (2010): 57-67.
Non Patent Literature 3: Zhong, Zhendong A et al. "Regulation of Wnt receptor activity: Implications for therapeutic development in colon cancer. " The Journal of biological chemistry vol. 296 (2021): 100782. doi:10.1016/j.jbc.2021.100782
Non Patent Literature 4: Phull, Manjinder Singh et al. "A perspective on medicinal chemistry approaches towards adenomatous polyposis coli and Wnt signal based colorectal cancer inhibitors. " European journal of medicinal chemistry vol. 212 (2021): 113149. doi:10.1016/j.ejmech.2020.113149

### SUMMARY OF INVENTION

### Technical Problem

However, the Wnt/β-catenin pathway has various physiological activities in living bodies. Therefore, it is difficult to target the Wnt/β-catenin pathway for treating, because various physiological activities in the living bodies are also inhibited when the Wnt/β-catenin pathway is blocked.

The present inventors have found that prorenin receptors contribute to the activation of the Wnt/β-catenin pathway, and the Wnt/β-catenin pathway can be inhibited by using antibodies against the prorenin receptors (PRR). Thus, the present inventors have conceived of treating diseases arising in connection with the Wnt/ β-catenin pathway by using the PRR peptide. Further, the present inventors examined whether the 200-213th peptide of PRR (PRRO) can be used to obtain an antitumor effect in a cancer-bearing model mouse. However, a sufficient antitumor effect was not obtained in a mouse administered with the PRR0. This was presumed to be due to an insufficient ability for inducing antibodies against the PRR.

Hence, it is an object of the present invention to provide a PRR peptide with enhanced inducibility of antibodies against prorenin receptors.

### Solution to Problem

To achieve the above object, the presnet dislosure provides a prorenin receptor peptide, including a polypeptide of following (P1), (P2), or (P3):
(P1) a polypeptide consisting of an amino acid sequence of any one of SEQ ID NO: 1-3;
(P2) a polypeptide consisting of an amino acid sequence of any one of SEQ ID NO: 1-3 in which one or two or three amino acids are deleted, substituted, or added, or
(P3) a polynucleotide consisting of an amino acid sequence having 80% or higher identity to an amino acid sequence of any one of SEQ ID NO: 1-3.

The present disclosure also provides a conjugate, including:
an antigenic peptide, and
a carrier protein, wherein
the antigenic peptide binds to the carrier protein, and
the antigenic peptide includes the prorenin receptor peptide of the present disclosure.

The present disclosure also provides a nucleic acid encoding the prorenin receptor peptide of the present disclosure.

The present disclosure also provides an expression vector including the nucleic acid of the present disclosure.

The present disclosure also provides a pharmaceutical composition, including:
at least one component selected from the group consisting of the prorenin receptor peptide of the present disclosure, the conjugate of the present disclosure, the nucleic acid of the present disclosure, and the expression vector of the present disclosure, and
a pharmaceutically acceptable carrier.

### Advantageous Effects of Invention

The PRR peptide of the present disclosure has an enhanced inducibility of antibodies against prorenin receptors.

### BRIEF DESCRIPTION OF DRAWINGS

[FIG. 1] FIG. 1 shows graphs of the antibody titer of the prorenin receptor vaccine PRR0 in Example 1.
[FIG. 2] FIG. 2 shows images of mice 10 weeks after the first inoculation of the prorenin receptor vaccine PRR0 in Example 1.
[FIG. 3] FIG. 3 is a graph showing the antibody titer of the prorenin receptor vaccines PRR1 to PRR4 in Example 1.
[FIG. 4] FIG. 4 is a graph showing the antitumor effect of the prorenin receptor vaccines PRR1 to PRR4 in Example 1.
[FIG. 5] FIG. 5 is a graph showing the antitumor effect of the prorenin receptor vaccines PRR2 and PRR3 in Example 1.
[FIG. 6] FIG. 6 shows images of stained β-catenin in colon tissue in Example 1.
[FIG. 7] FIG. 7 shows graphs of the antibody titer of the prorenin receptor vaccines PRR2 and PRR3 under short-term observations in Example 1.
[FIG. 8] FIG. 8 shows graphs of the antibody titer of the prorenin receptor vaccines PRR2 and PRR3 under long-term observations in Example 1.
[FIG. 9] FIG. 9 is a graph showing the antibody titer of CRM197-PRR2 in the mice in Example 2.
[FIG. 10] FIG. 10 is a graph showing the antibody titer of CRM197-PRR2 in cynomolgus monkeys in Example 2.
[FIGs. 11A and 11B] FIGs. 11A and 11B show a graph of the activity of β-catenin in HEK293 cells in Example 3.
[FIGs. 12A to 12C] FIGs. 12A to 12C show graphs of the inhibitory effect against polyp formation in adenomatosis polyposis model mice in Example 3.
[FIG. 13] FIG. 13 is a graph showing the inhibitory effect against polyp formation in adenomatosis polyposis model mice in Example 3.
[FIGs. 14A and 14B] FIGs. 14A and 14B are images of stained ATP6ap2 or active β-catenin in the intestine in Example 3.
[FIGs. 15A to 15F] FIGs. 15A to 15F show graphs of the measurements of adverse reactions in adenomatosis polyposis model mice and normal mice in Example 3.
[FIG. 16] FIG. 16 shows graphs of the survival rates of adenomatosis polyposis model mice in Example 3.
[FIG. 17] FIG. 17 is a graph showing the antibody titer of the vaccine PRR2 in Example 4.
[FIGs. 18A and 18B] FIGs. 18A and 18B are graphs showing the weight shift after administering PRR2 in Example 4.

### DESCRIPTION OF EMBODIMENTS

Hereinafter, the present invention will be described in more detail with reference to examples. Regarding the descriptions of the invention, reference can be made to each other unless otherwise stated.

### <Definition>

As used herein, a "protein" and a "polypeptide" refer to a polymer of a peptide composed of a unmodified amino acid (natural amino acid), a modified amino acid, and/or an artificial amino acid. The shape of the polymer may be, for example, liner, branched, cyclic, or the like.

As used herein, a "prorenin receptor" refers to a single-pass transmembrane protein identified as a factor constituting a renin-angiotensin system, which is a protein that functions as a prorenin receptor. The prorenin receptor is also referred to as ATP6AP2. The prorenin receptor is presumed to indirectly enhance the activity of a Wnt/β-catenin system.

As used herein, a "conjugate" refers to a compound in which two or more peptides with different amino acid sequences are covalently bonded. The peptides with different amino acid sequences are, for example, antigenic peptides, carrier peptides, or carrier proteins. The antigenic peptides are, for example, peptides for inducing antibodies by inducing immune response and activation of lymphocytes and the like. The carrier peptides or the carrier proteins are, for example, peptides or proteins which impart immunogenicity to the antigenic peptides, or enhance the immunogenicity of the antigenic peptides. The carrier peptides and the carrier proteins may also be referred to as, for example, peptide carriers and protein carriers.

As used herein, a "Wnt/β-catenin pathway" refers to a signal transduction pathway in which Wnt activates β-catenin via a Wnt receptor (Frizzled). The Wnt/β-catenin pathway contributes to induction of diseases, for example in cancer such as colorectal cancer, through abnormal accumulation of β-catenin, inactivation of gene expression of Wnt inhibitors, and like.

As used herein, "inhibition of activity" refers to a change to the state where the activity of a subject is inhibited, or the inhibited state thereof. When the "inhibition of activity" is used in combination with a specific protein or a signal pathway via the specific protein, the "inhibition of activity" refers to a change to the state where the function of the specific protein or the signal pathway via the specific protein is inhibited, or the inhibited state thereof.

As used herein, "sarcopenia or frail" refers to a disease caused by impaired function of muscle tissue.

As used herein, "positive" refers to detection of a signal or the like higher than that of a negative control cell which does not express the antigen, or a negative control reaction using an antibody which does not react with the antigen, by an analysis method such as flow cytometry using an antigen-antibody reaction. As used herein, "negative" refers to detection of a signal or the like equivalent to or lower than that of the negative control cell which does not express the antigen, or the negative control reaction using the antibody which does not react with the antigen.

As used herein, "treatment" refers to therapeutic treatment and/or prophylactic treatment. As used herein, "cure" refers to medical treatment, recovery, protection, suppression, remission, or improvement of a disease, a symptom, or a disorder, or to stopping, suppressing, reducing, or delaying progression of a disease, a symptom, or a disorder. As used herein, "prevention" refers to decreasing the possibility of development of a disease or a symptom, or delaying the development of a disease or a symptom. The "cure" may be, for example, cure for a subject (patient) who develops a disease of interest, or for a model animal who develops the disease of interest.

As used herein, a "subject" refers to a cell, tissue or an organ of an animal or derived from an animal, including a human in particular. The animal refers to a human and a non-human animal. Examples of the non-human animal include mammals such as a mouse, a rat, a rabbit, a dog, a cat, a cow, a horse, a pig, a monkey, a dolphin, and a sea lion.

As used herein, "nucleic acid" refers to deoxyribonucleotide (DNA), ribonucleotide (RNA), and/or a polymer of a modified nucleotide. As used herein, when "nucleic acid" is used in combination with a specific protein, the "nucleic acid" refers to a polymer of a nucleotide encoding an amino acid sequence of a protein. Examples of the nucleic acid include genomic DNA, cDNA, and mRNA. The nucleic acid may be, for example, single-stranded or double-stranded. The nucleic acid can be interchangeably replaced with a "polynucleotide" or a "nucleic acid molecule".

As used herein, a "host" refers to a cell and/or an individual into which foreign nucleic acid is introduced. When the host is a cell, the host may also be referred to as a host cell.

As used herein, a "vector" and an "expression vector" refer to a recombinant plasmid or a virus which includes nucleic acid to be delivered to a host or a host cell *in vitro* or *in vivo.* The "vector" and the "expression vector" may be a viral vector or a non-viral vector.

As used herein, a "transformant" refers to a host into which foreign nucleic acid is introduced.

As used herein, "isolation" or "isolated" refers to an identified and isolated state and/or a state being recovered from a component in its natural state. The "isolation" or "isolated" can be carried out, for example through at least one purification process.

Hereinafter the present disclosure will be described with reference to example embodiments. However, the present disclosure is by no means limited thereto, and can be carried out with an arbitrary change. Regarding the descriptions of the invention, reference can be made to each other unless otherwise stated. As used herein, the expression "-" is intended to include numerical or physical values before and after the expression "-". Also, as used herein, the expression "A and/or B" is intended to include "only A", "only B", and "both A and B".

### <Prorenin Receptor Polypeptide>

In some embodiments, the present disclosure provides a polypeptide with enhanced inducibility of antibodies against prorenin receptors. The prorenin receptor peptide of the present disclosure includes the following polypeptide (P1), (P2), or (P3):
(P1) A polypeptide consisting of an amino acid sequence of any one of SEQ ID NO: 1-3;
(P2) A polypeptide consisting of an amino acid sequence of any one of SEQ ID NO: 1-3 in which one or more amino acids are deleted, substituted, or added, or
(P3) A polynucleotide consisting of an amino acid sequence having 70% or more of identity to an amino acid sequence of any one of SEQ ID NO: 1-3.

As a result of intensive studies, the present inventors have found that an antibody against the prorenin receptor (PRR) exerts an antitumor effect or the like by regulating the activity of the Wnt/β-catenin pathway. As a result of further studies, the present inventors have found that the antibody against PRR can be induced *in vivo* by administering a peptide having a specific amino acid sequence in PRR, whereby an antitumor effect and a suppression effect on muscle mass reduction can be obtained similarly to the antibody against PRR, and reached the completion of the present disclosure. It is presumed that the PRR peptide of the present disclosure exhibits an antitumor effect and a suppression effect on muscle mass reduction by inducing the production of antibodies which indirectly regulate the activity of the Wnt/β-catenin pathway in a living body administered with the PRR peptide. However, this presumption by no means limits the present disclosure. Thus, the PRR peptide of the present disclosure can be suitably used, for example, for the treatment of tumors (e.g., benign tumors such as familial adenomatous polyposis) and sarcopenia or frail.

The origin of the prorenin receptor is not particularly limited, and can be appropriately set depending on, for example, the type of the subject. Examples of the origin include humans and non-human animals except humans. Examples of the non-human animals include mammals such as mice, rats, dogs, monkeys, rabbits, sheep, and horses. Reference for the prorenin receptors derived from various animals can be made in information registered in an existing database, for example. As a specific example, the prorenin receptor derived from humans may be, as cDNAfor example, 103- 1155th region (including a termination codon) in the following base sequence (SEQ ID NO: 4) registered under NCBI Accession NO. NM_005765, and as a protein for example, the following amino acid sequence (SEQ ID NO: 5) registered under NCBI Accession NO. NP_005756. The base sequence of SEQ ID NO: 4 is a sequence encoding the amino acid sequence of SEQ ID NO: 5.
Human prorenin receptor cDNA (SEQ ID NO: 4) 
ctggacgagtccgagcgcgtcacctcctcacgctgcggctgtcgcccgtgtcccgccggcccgttccgtgtcgccccgcagtgctgcggc
cgccgcggcaccatggctgtgtttgtcgtgctcctggcgttggtggcgggtgttttggggaacgagtttagtatattaaaatcaccagggtctg
ttgttttccgaaatggaaattggcctataccaggagagcggatcccagacgtggctgcattgtccatgggcttctctgtgaaagaagacctttc
ttggccaggactcgcagtgggtaacctgtttcatcgtcctcgggctaccgtcatggtgatggtgaagggagtgaacaaactggctctacccc
caggcagtgtcatttcgtaccctttggagaatgcagttccttttagtcttgacagtgttgcaaattccattcactccttattttctgaggaaactcct
gttgttttgcagttggctcccagtgaggaaagagtgtatatggtagggaaggcaaactcagtgtttgaagacctttcagtcaccttgcgccag
ctccgtaatcgcctgtttcaagaaaactctgttctcagttcactccccctcaattctctgagtaggaacaatgaagttgacctgctctttctttctga
actgcaagtgctacatgatatttcaagcttgctgtctcgtcataagcatctagccaaggatcattctcctgatttatattcactggagctggcagg
tttggatgaaattgggaagcgttatggggaagactctgaacaattcagagatgcttctaagatccttgttgacgctctgcaaaagtttgcagat
gacatgtacagtctttatggtgggaatgcagtggtagagttagtcactgtcaagtcatttgacacctccctcattaggaagacaaggactatcct
tgaggcaaaacaagcgaagaacccagcaagtccctataaccttgcatataagtataattttgaatattccgtggttttcaacatggtactttggat
aatgatcgccttggccttggctgtgattatcacctcttacaatatttggaacatggatcctggatatgatagcatcatttataggatgacaaacca
gaagattcgaatggattgaatgttacctgtgccagaattagaaaagggggttggaaattggctgttttgttaaaatatatcttttagtgtgctttaa
agtagatagtatactttacatttataaaaaaaaatcaaattttgttctttattttgtgtgtgcctgtgatgtttttctagagtgaattatagtattgacgtg
aatcccactgtggtatagattccataatatgcttgaatattatgatatagccatttaataacattgatttcattctgtttaatgaatttggaaatatgca
ctgaaagaaatgtaaaacatttagaatagctcgtgttatggaaaaaagtgcactgaatttattagacaaacttacgaatgcttaacttctttacac
agcataggtgaaaatcatatttgggctattgtatactatgaacaatttgtaaatgtcttaatttgatgtaaataactctgaaacaagagaaaaggtt
tttaacttagagtagccctaaaatatggatgtgcttatataatcgcttagttttggaactgtatctgagtaacagaggacagctgttttttaaccctc
ttctgcaagtttgttgacctacatgggctaatatggatactaaaaatactacattgatctaagaagaaactagccttgtggagtatatagatgcttt
tcattatacacacaaaaatccctgagggacattttgaggcatgaatataaaacatttttatttcagtaacttttccccctgtgtaagttactatggttt
gtggtacaacttcattctatagaatattaagtggaagtgggtgaattctactttttatgttggagtggaccaatgtctatcaagagtgacaaataa
agttaatgatgattccaaaaaaaaaa
Prorenin receptor (SEQ ID NO: 5)

MAVFVVLLALVAGVLGNEFSILKSPGSVVFRNGNWPIPGERIPDVAALSMGFSVKEDLSW
PGLAVGNLFHRPRATVMVMVKGVNKLALPPGSVISYPLENAVPFSLDSVANSIHSLFSEE
TPVVLQLAPSEERVYMVGKANSVFEDLSVTLRQLRKRLFQENSVLSSLPLNSHSRNNEV

DLLFLSELQVLHDISSLLSRHKHLAKDHSPDLYSLELAGLDEIGKRYGEDSEQFRDASKIL
VDALQKFADDMYSLYGGNAVVELVTVKSFDTSLIRKTRTILEAKQAKNPASPYNLAYKY
NFEYSVVFNMVLWIMIALALAVIITSYNIWNMDPGYDSIIYRMTNQKIRMD

In the (P1), the polypeptide consisting of the amino acid sequences of SEQ ID NO: 1 to 3 is a polypeptide derived from a human prorenin receptor. The amino acid sequence of SEQ ID NO: 1 corresponds to the 198-211th amino acid sequences in the amino acid sequence of the human prorenin receptor (SEQ ID NO: 5). The amino acid sequence of SEQ ID NO: 2 corresponds to the 223-236th amino acid sequences in the amino acid sequence of the human prorenin receptor (SEQ ID NO: 5). The amino acid sequence of SEQ ID NO: 3 corresponds to the 221-230th amino acid sequences in the amino acid sequence of the human prorenin receptor (SEQ ID NO: 5).

PRR1 peptide (SEQ ID NO: 1)
   SRHKHLAKDHSPDL
PRR2 peptide (SEQ ID NO: 2)
   GKRYGEDSEQFRDA
PRR3 peptide (SEQ ID NO: 3)
   EIGKRYGEDS

In the polypeptide of the (P2), "one or more" is preferably set, for example, within a range where the polypeptide of the (P2) is capable of inducing the production of antibodies against PRR, for example, when a conjugate is formed with a carrier protein. In the amino acid sequence of the (P1), "one or more" is set to, for example, 1-3, 1 or 2, or 1. In the present disclosure, the numerical range of the number discloses, for example, all positive integers belonging to the range (same applies hereinafter).

In the polypeptide of the (P2), the substitution is preferably a conservative substitution. The conservative substitution refers to a substitution of an amino acid residue with an amino acid residue having a similar side chain. The conservative substitution may be, for example, a substitution between amino acid residues having basic side chains, such as lysine, arginine, and histidine; a substitution between amino acid residues having acidic side chains, such as aspartic acid and glutamic acid; a substitution between amino acid residues having polar noncharged side chains, such as glycine, asparagine, glutamine, serine, threonine, tyrosine, and cysteine; a substitution between amino acid residues having nonpolar side chains, such as alanine, valine, leucine, isoleucine, proline, phenylalanine, methionine, and tryptophan; a substitution between amino acid residues having β-branched side chains, such as threonine, valine, and isoleucine, and a substitution between amino acid residues having aromatic side chains, such as tyrosine, phenylalanine, tryptophan, and histidine.

In the (P3), "identity" is preferably set, for example, within a range where the polypeptide of the (P3) is capable of inducing the production of antibodies against PRR, for example, when forming a conjugate with a carrier protein. The "identity" is, for example, 70% or more, 80% or more, 85% or more, 90% or more, 95% or more, 96% or more, 97% or more, 98% or more, or 99% or more to the amino acid sequence of the (P1). The "identity" can be calculated, for example, by a using default parameter in Homology Algorithm BLAST (http://www.ncbi.nlm.nih.govBLAST/) of the National Center for Biotechnology Information (NCBI) (same applies hereinafter).

The PRR peptide may include, for example, a linker amino acid or a linker peptide. The linker amino acid or the linker peptide is an amino acid or a peptide added for binding a carrier protein or a carrier peptide to an N-terminus and/or a C-terminus. In the PRR peptide, the N-terminus, the C-terminus, the main chain of the amino acid, and/or the side chain of the amino acid may be modified. The linker amino acid is, for example, cysteine.

The PRR peptide of the present disclosure can be used, for example, in combination with a carrier peptide or a carrier protein to induce antibodies against the PRR.

### <Conjugate>

In another embodiment, the present disclosure provides a conjugate with enhanced inducibility of antibodies against prorenin receptors. The present disclosure includes an antigenic peptide and a carrier protein, wherein the antigenic peptide binds to the carrier protein, and the antigenic peptide includes the prorenin receptor peptide.

In the conjugate, the carrier protein may be any protein capable of imparting or enhancing the immunogenicity of the PRR peptide. Examples of the carrier protein include a diphtheria toxin (DT) or a mutant of a diphtheria toxin, a tetanus toxin (TT) or a fragment C of a tetanus toxin, a keyhole limpet hemocyanin (KLH), or a functional equivalent of these. The functional equivalent is a mutant in which mutation is introduced into an amino acid sequence within a range where a function as the carrier protein exists.

The mutant of the diphtheria toxin may be, for example, a detoxified diphtheria toxin CRM₁₉₇, the A-chain of CRM₁₉₇ (CN103495161), CRM176, CRM228, CRM45 (Uchida et al. (1973), J.Biol.Chem. 218:3838-3844), CRM9, CRM102, CRM103, CRM107, or the like.

The carrier protein is preferably CRM₁₉₇ because of being able to be suitably used, for example, as a vaccine composition. The CRM₁₉₇ may be, as a protein, for example, a protein or a functional equivalent thereof in which the 52nd glycine of the following amino acid sequence (SEQ ID NO: 6) registered under Uniprot Accession NO: Q5PY51 is substituted with glutamic acid.
CRM₁₉₇ (SEQ ID NO: 6)

MGADDVVDSSKSFVMENFSSYHGTKPGYVDSIQKGIQKPKSGTQGNYDDDWKEFYSTD
NKYDAAGYSVDNENPLSGKAGGVVKVTYPGLTKVLALKVDNAETIKKELGLSLTEPLM
EQVGTEEFIKRFGDGASRVVLSLPFAEGSSSVEYINNWEQAKALSVELEINFETRGKRGQ
DAMYEYMAQACAGNRVRRSVGSSLSCINLDWDVIRDKTKTKIESLKEHGPIKNKMSESP
NKTVSEEKAKQYLEEFHQTALEHPELSELKTVTGTNPVFAGANYAAWAVNVAQVIDSET
ADNLEKTTAALSILPGIGSVMGIADGAVHHNTEEIVAQSIALSSLMVAQAIPLVGELVDIGF
AAYNFVESIINLFQVVHNSYNRPAYSPGHKTQPFLHDGYAVSWNTVEDSIIRTGFQGESG
HDIKITAENTPLPIAGVLLPTIPGKLDVNKSKTHISVNGRKIRMRCRAIDGDVTFCRPKSP
VYVGNGVHANLHVAFHRSSSEKIHSNEISSDSIGVLGYQKTVDHTKVNSKLSLFFEIKS

In the conjugate, the antigenic peptide and the carrier protein are covalently bonded to each other. The binding position to the carrier protein in the antigenic peptide is, for example, an N-terminus, a C-terminus, and/or a side chain of an amino acid of the antigenic peptide, and preferably the N-terminus (amino group) and/or the C-terminus (carboxyl group) of the antigenic peptide.

The binding position to the antigenic peptide in the carrier protein is, for example, an N-terminus, a C-terminus, and/or a side chain of an amino acid of the carrier protein, and preferably the amino acid side chain. Examples of the amino acid side chain include a side chain of a cysteine residue (thiol group), a side chain of a lysine residue, an arginine residue, a glutamine residue, or a serine residue (amino group), and a side chain of glutamic acid or aspartic acid (carboxyl group).

In the conjugate, the antigenic peptide and the carrier protein are directly or indirectly bonded to each other. The direct binding means that the antigenic peptide is directly bonded to an amino acid of the carrier protein. On the other hand, the indirect binding means that the antigenic peptide is bonded to the carrier protein via a linker peptide (peptide linker).

In the conjugate, the antigenic peptide is preferably bonded to, for example, the cysteine residue of the carrier protein. The binding between the antigenic peptide and the carrier protein can be set based on, for example, a reactive group of the antigenic peptide and a reactive group of the carrier protein used for the binding. The antigen peptide and the carrier protein may be bonded, for example, by directly reacting the reactive group of the antigen peptide and the reactive group of the carrier protein to each other, or may be bonded by reacting a cross-linking agent with the antigen peptide and the carrier protein. As a specific example, when binding the amino group of the antigenic peptide and a side chain of the cysteine residue (thiol group) of the carrier protein to each other, the antigenic peptide and the carrier protein can be bonded by crosslinking using, for example, a crosslinking agent having a different reactive group, more specifically, a crosslinking agent having a reactive group having reactivity with an amino group and a reactive group having reactivity with a thiol group. Examples of the crosslinking agent include N-ε-malemidocaproyl-oxysuccinimide ester (EMCS), N-ε-maleimidocaproyl-oxysulfosuccinimide ester (Sulfo-EMCS), succinimidyl 4-(N-maleimidomethyl)cyclohexane-1-carboxylate (SMCC), sulfosuccinimidyl 4-(N-maleimidomethyl)cyclohexane-1-carboxylate (Sulfo-SMCC), N-α-maleimidoacet-oxysuccinimide ester (AMAS), N-β-maleimidopropyl-oxysuccinimide ester (BMPS), and N-γ-maleimidobutyryl-oxysuccinimide ester (GMBS).

In the conjugate, the ratio of the carrier protein to the antigenic peptide (molecule number ratio of carrier protein: antigenic peptide) may be, for example, 1:1-1:100, or 1:1-1:10.

The conjugate is preferred to have ability to induce formation of an antibody capable of inhibiting the activity of the Wnt/β-catenin pathway in an activity assay of the Wnt/β-catenin pathway, when being administered to a subject. The activity assay of the Wnt/β-catenin pathway is, for example, a system for evaluating the degree of activation of the β-catenin pathway *in vitro* using a plasma or a serum derived from the subject administered with the conjugate of interest, and a reporter cell, for example, in accordance with Example 3(1) described later. The reporter cell is a cell in which a reporter (e.g., luciferase) is detected when the β-catenin pathway is activated.

The inducibility can be measured, for example, using a conjugate to be measured (measurement object), by administering (inoculating) the measurement object to a subject, and evaluating an activity assay of a Wnt/β-catenin pathway in a plasma or a serum derived from the subject. Specifically, the measurement object is administered to the subject, for example, in the same manner as in Examples 1 and 2 described later, by arbitrarily being mixed with an immunostimulant. The administration is preferably carried out twice. Blood is then collected from the subject, for example, 2-4 weeks after the last administration, and a serum or a plasma is isolated from the blood. The inducibility can then be evaluated, for example, by performing the activity assay of the Wnt/β-catenin pathway with the plasma or the serum, and comparing with the activity of the Wnt/β-catenin pathway of a plasma or a serum derived from a subject administered with saline (control).

The conjugate has, for example, inducibility of formation of antibodies capable of inhibiting the activity of the Wnt/β-catenin pathway 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, on the basis of the activity (100%) of the Wnt/β-catenin pathway of the control in the activity assay of the Wnt/β-catenin pathway.

The conjugate is used, for example, for treatment of a tumor. The tumor may be a benign tumor or a malignant tumor (cancer). The tumor may be, for example, a prorenin receptor positive tumor. Examples of the tumor include colorectal cancer, pancreatic cancer, and brain cancer. The tumor may be, for example, a benign tumor or familial adenomatous polyposis.

The conjugate can be suitably used, for example, for treatment of familial adenomatous polyposis causing complications. Examples of the complications of familial adenomatous polyposis include gastrointestinal benign tumor multiple neoplasia, Gardner's syndrome, Turcot's syndrome, and/or Desmoid Fibromatosis.

The conjugate of the present disclosure may be used, for example, *in vitro* or *in vivo.* The conjugate of the present disclosure can be used, for example, as a research reagent or as pharmaceuticals. In the former case, the conjugate of the present disclosure can also be referred to as a test reagent or a test kit.

The subject to be administered with the conjugate of the present disclosure is not particularly limited. When using the conjugate of the present disclosure *in vivo,* for example, the aforementioned exemplification can be applied to the subject (administration subject). When using the conjugate of the present disclosure *in vitro,* the administration subject may be, for example, a cell, a tissue, an organ, or the like. The cell may be, for example, a cell collected from a living body, a cultured cell, or the like. The tissue or the organ may be, for example, tissue (living tissue) or an organ collected from a living body, or the like. Examples of the cell include an immune cell such as a T cell, a B cell, a NK cell, and a dendritic cell.

When using the conjugate of the present invention *in vivo,* the administration subject may be a healthy subject who does not have a tumor, a subject who may have a tumor, or a patient who has a tumor, and preferably a subject who is desired to receive a treatment. Also, when the administration subject has familial adenomatous polyposis, the administration subject may be a patient who does not have a complication of the familial adenomatous polyposis, a patient who may have a complication of the familial adenomatous polyposis, or a patient who has a complication of the familial adenomatous polyposis, and preferably a subject who is desired to receive a treatment. Further, the administration subject may be a healthy subject who does not have sarcopenia or frail, a subject who may have sarcopenia or frail, or a patient who has sarcopenia or frail, and preferably a subject who is desired to receive a treatment.

The use conditions (administration conditions) of the conjugate of the present disclosure are not particularly limited, and for example, the administration form, the administration period, the dosage, and the like can be appropriately set depending on the type of an active component (e.g., protein, peptide, VLP, virus, and nucleic acid) in the conjugate, the type of the administration subject, and the like.

Examples of the method for administering the conjugate of the present disclosure include intracerebral administration, intrathecal administration, intramuscular administration, subcutaneous administration, and intravenous administration. Among these methods, for example, intramuscular administration or subcutaneous administration is preferable as the administration can be carried out safely and stably regardless of the skill of the administering person.

The dosage of the conjugate of the present disclosure may be, any amount enough for inducing antibodies against the prorenin receptor compared with the subject without the administration, when being administered to the administration subject, that is an effective dosage (therapeutically effective dosage). The dosage can be appropriately determined depending on, for example, the age, weight, symptoms, and the like of the administration subject.

The number of administration times of the conjugate of the present disclosure is one or several times. The "several times" refers to, for example, 2 times, 3 times, 4 times, 5 times, 5 times or more. The number of administration times may be appropriately determined while confirming the therapeutic effect in the administration subject. In the case of the several administrations, the administration interval can be appropriately determined while confirming the treatment effect in the administration subject, and for example, once in a day, once in a week, once in two weeks, once in a month, once in three months, once in six months, and the like.

The conjugate of the present disclosure is capable of preventing or alleviating at least one symptom caused by a tumor in the administration subject. Examples of the symptom of tumor onset include general malaise, anorexia, and weight loss. Examples of the symptom of the familial adenomatous polyposis include increase in the number of polyps larger than the diameter of 5mm which need to be endoscopically removed. The conjugate of the present disclosure is capable of preventing or alleviating at least one symptom associated with a tumor. The alleviation of the symptom can be subjectively or objectively evaluated, for example. Specific examples of the evaluation include self-evaluation by the administration subject; evaluation by a physician; QOL (Quality of Life) evaluation; delay in the progression of the symptom of tumor onset, or evaluation of alleviation in the severity of the symptom of tumor onset. The objective evaluation may be an evaluation in an animal or an evaluation in a human.

When using the conjugate of the present disclosure *in vivo,* the administration subject may be a healthy subject who is not suffering from sarcopenia or frail, a subject who may be suffering from sarcopenia or frail, or a patient suffering from sarcopenia or frail.

The conjugate of the present disclosure is capable of preventing or alleviating at least one symptom caused by sarcopenia or frail in an administration subject. Examples of the symptom of sarcopenia or frail include decrease in body weight, skeletal muscle mass, muscle strength (grip strength), or physical function (walking speed). The conjugate of the present disclosure is capable of preventing or alleviating at least one symptom associated with sarcopenia or frail. The alleviation of the symptom can be subjectively or objectively evaluated, for example. Specific examples of the evaluation include self-evaluation by the administration subject; evaluation by a physician; QOL (Quality of Life) evaluation; delay in the progression of the symptom of sarcopenia or frail, and evaluation of alleviation in the severity of the symptom of sarcopenia or frail. The objective evaluation may be an evaluation in an animal or an evaluation in a human.

The conjugate of the present disclosure includes the prorenin receptor peptide, and thus can induce antibodies against PRR. Therefore, the conjugate of the present disclosure can be suitably used for the treatment of cancer and the treatment of sarcopenia or frail.

### <Nucleic Acid>

In another embodiment, the present disclosure provides a nucleic acid encoding the PRR peptide. The nucleic acid of the present disclosure encodes the prorenin receptor peptide of the present disclosure.

The nucleic acid may be composed of either or both of a deoxynucleotide residue and a ribonucleotide residue. The nucleic acid may be composed of either or both of a natural nucleic acid residue and a non-natural nucleic acid residue. Specific examples of the nucleic acid include DNA, RNA, and/or DNA/RNA composed of a natural nucleic acid residue and/or a non-natural nucleic acid residue. The non-natural nucleic acid residue may be, for example, a modified nucleotide residue or a modified ribonucleotide residue obtained by modifying a base, a sugar residue, or a sugar phosphate backbone in a nucleotide residue. When the sugar residue is modified, the non-natural nucleic acid residue may be, for example, cEt (constrained ethyl bicyclic nucleic acid, manufactured by Ionis Pharmaceuticals, Inc.), LNA^{®}((trademark), Locked Nucleic Acid), ENA^{®} ((registered trademark), 2'-O,4'-C-Ethylenebridged Nucleic Acid), and the like. The nucleic acid may have a 5' cap at the 5' end, for example.

The nucleic acid may be a single-stranded nucleic acid molecule or a double-stranded nucleic acid molecule.

The nucleic acid of the present disclosure can be designed by replacing the corresponding codon on the basis of the amino acid sequence of the PRR peptide of the present disclosure. The base sequence of the nucleic acid of the present disclosure may be, for example, codon-optimized.

In the present disclosure, various nucleic acids and prorenin receptor peptides can be synthesized by, for example, genetic engineering techniques or organic synthetic techniques, and can also be referred to as synthetic DNA such as cDNA, or synthetic RNA.

The nucleic acid of the present disclosure encodes the PRR peptide, and thus can be suitably used for synthesizing the PRR peptide. The nucleic acid of the present disclosure encodes the PRR peptide, and thus can induce antibodies against the PRR, when being used as an active component of a vaccine.

### <Expression Vector>

In another embodiment, the present disclosure provides an expression vector which can be used for synthesis or expression of a prorenin receptor peptide. The expression vector of the present disclosure includes the nucleic acid of the present disclosure. The expression vector of the present disclosure allows suitably producing the prorenin receptor peptide of the present disclosure with a genetic engineering technique.

The expression vector of the present disclosure is, for example, an expression vector into which the nucleic acid of the present disclosure is inserted. It may also be said that the nucleic acid is functionally linked to the expression vector. It can be also said that the expression vector is, for example, an expression vector to which the nucleic acid is functionally linked. The expression vector refers to, for example, a nucleic acid molecule capable of transporting an inserted gene into a target such as a cell.

The configuration of the expression vector is not particularly limited as long as it includes a polynucleotide encoding a prorenin receptor peptide, for example, such that the prorenin receptor peptide encoded by the polynucleotide of the nucleic acid of the present disclosure can be expressed.

The expression vector can be prepared, for example, by inserting a polynucleotide encoding a prorenin receptor peptide, that is, the nucleic acid of the present disclosure, into a vector serving as a framework (hereinafter, also referred to as a "basic vector"). A type of the expression vector is not particularly limited, and can be appropriately determined depending on, for example, a type of the host. Specifically, when synthesizing the expression vector by a genetic engineering technique, the nucleic acid encoding the prorenin receptor peptide, for example, is firstly designed to synthesize the expression vector. The design and the synthesis can be performed by a PCR method using, for example, a vector including the nucleic acid encoding the prorenin receptor peptide as a template, and a primer designed to synthesize a desired nucleic acid region. Then, the obtained nucleic acid is linked to an appropriate vector to obtain a recombinant vector for protein expression (expression vector), and this recombinant vector is introduced into a host so that a gene of interest can be expressed, thereby a transformant can be obtained (Sambrook J.et al., Molecular Cloning, A Laboratory Manual (4th edition) (Cold Spring Harbor Laboratory Press (2012)).

The host used for transformation is not particularly limited as long as being capable of expressing the nucleic acid of interest, and examples thereof include a microorganism, an animal cell, an insect cell, a non-human host such as cultured cells of these, an isolated human cell or a cultured cell thereof, and a mammalian cell. When administering the expression vector to a subject, the host is, for example, a cell of the administration subject. Examples of the prokaryote include bacteria such as *Escherichia* genus such as *Escherichia coli*, and bacteria such as *Pseudomonas* genus such as *Pseudomonas putida.* Examples of the eucaryote include yeast such as *Saccharomyces cerevisiae.* Examples of the animal cell include a COS cell, and a CHO cell. Examples of the insect cell include Sf9, and Sf21.

Examples of the expression vector include a viral vector and a non-viral vector. Examples of the viral vector include a baculovirus; a poxvirus such as vaccinia virus, an Avi poxvirus, a canarypox virus, a fowl pox virus, a raccoon pox virus, and a swine pox virus; an adenovirus such as canine adenovirus; an adeno-associated virus; a herpes virus, and a retrovirus. When adopting a heat shock method as the introduction method to transform the host, the expression vector may be, for example, a binary vector or the like. Examples of the expression vector include pETDuet-1, pQE-80L, and pUCP26Km. When transforming bacteria such as *Escherichia coli*, the expression vector may be, for example, a pETDuet-1 vector (manufactured by Novagen), pQE-80L (manufactured by QIAGEN), pBR322, pB325, pAT153, pUC8, or the like. When transforming the yeast, the expression vector may be, for example, pYepSec1, pMFa, pYES2, or the like. When transforming the insect cell, the expression vector may be, for example, pAc, pVL, or the like. When transforming the mammalian cell, the expression vector may be, for example, pCDM8, pMT2PC, or the like.

The expression vector is preferred to have a regulatory sequence which regulates, for example, the expression of the polynucleotide encoding the prorenin receptor peptide, and the expression of the prorenin receptor peptide of the present disclosure encoded by the polynucleotide of the prorenin receptor peptide. Examples of the regulatory sequence include a promoter, a terminator, an enhancer, a polyadenylation signal sequence, and a replication origin sequence (ori). In the expression vector, the arrangement of the regulatory sequences is not particularly limited. In the expression vector, the regulatory sequence may be arranged, for example, such that the expression of the polynucleotide encoding the prorenin receptor peptide and the expression of the polypeptide encoded by the polynucleotide can be functionally regulated, for example, and may be arranged according to a known method. As the regulatory sequence, for example, a sequence previously present in the expression vector may be used, the regulatory sequence may further be inserted into the expression vector, or the regulatory sequence present in the basic vector may be replaced with another regulatory sequence.

### <Transformant and Method for Producing Transformant >

In another embodiment, the present disclosure provides a transformant capable of producing a prorenin receptor peptide, and a method for producing the same. The transformant of the present disclosure includes the nucleic acid encoding the prorenin receptor peptide of the present disclosure. The transformant of the present disclosure is characterized by including the nucleic acid encoding the prorenin receptor peptide of the present disclosure, and other configurations and conditions are not particularly limited. The transformant of the present disclosure allows suitably producing the prorenin receptor peptide of the present disclosure.

The method for producing the transformant according to the present disclosure includes introducing the nucleic acid of the present disclosure into a host. The method for producing the transformant according to the present disclosure is characterized in that the nucleic acid of the present disclosure is introduced into the host, and other processes and conditions are not particularly limited. The method for producing the transformant according to the present disclosure allows producing the transformant. The transformant and the method for producing the same according to the present disclosure can incorporate the description of the method for producing the prorenin receptor peptide, the conjugate, the nucleic acid, and the expression vector of the present disclosure.

In the transformant of the present disclosure, the description of the nucleic acid encoding the prorenin receptor peptide may incorporate that of the nucleic acid encoding the prorenin receptor peptide of the present disclosure. The expression vector of the present disclosure may be used as the nucleic acid of the present disclosure.

In the transformant of the present disclosure, the nucleic acid of the present disclosure is present as an exogenous molecule. Therefore, the transformant of the present disclosure can be produced, for example, by introducing the nucleic acid of the present disclosure into the host.

A method for introducing the nucleic acid is not particularly limited, and can be performed by a known method. The nucleic acid may be introduced, for example, by the expression vector. A method for introducing the expression vector into the host is not particularly limited, and can be performed by a known method. The introduction method can be appropriately set depending on, for example, a type of the host. Examples of the introduction method include an introduction method using a gene gun such as a particle gun, calcium phosphate transfection, polyethylene glycol transfection, lipofection using liposomes, electroporation, an nucleic acid introduction method using ultrasonic waves, DEAE-dextran transfection, a direct injection method using micro glass tubes or the like, hydrodynamic gene delivery, a method using cationic liposomes, a method using an introduction adjuvant, and a method of introduction via Agrobacterium. Examples of the liposomes include lipofectamine and cationic liposomes. Examples of the introduction adjuvant include atelocollagen, a nanoparticle, and a polymer. When the host is a microorganism, for example, a method of introduction via yeast or the like is preferable. The polynucleotide of the prorenin receptor peptide of the present disclosure may be introduced to the host by the expression vector of the present disclosure, for example.

### <Method for Producing PRR Peptide and Conjugate>

In another embodiment, the present disclosure provides a method for producing a prorenin receptor peptide. The method for producing the prorenin receptor peptide according to the present disclosure includes expressing the prorenin receptor peptide of the present disclosure. The method for producing the prorenin receptor peptide according to the present disclosure is characterized by including the expressing the prorenin receptor peptide of the present disclosure, and other processes and conditions are not particularly limited. The method for producing the prorenin receptor peptide according to the present disclosure allows producing the prorenin receptor peptide of the present disclosure.

The prorenin receptor peptide may be produced, for example, by a genetic engineering technique using a host such as yeast, or by a cell-free system, and a method well known to those skilled in the art may be used. For the production of the polypeptide, reference can be made to, for example, WO 2016/017037 A1(US 2016/0202251 A2).

When producing the prorenin receptor peptide by the genetic engineering technique, in the production method of the present disclosure, a transformant including the nucleic acid or the expression vector of the present disclosure may be prepared prior to the expressing the prorenin receptor peptide. In this case, the expressing the prorenin receptor peptide may include, for example, culturing the transformant and isolating the prorenin receptor peptide from the culture. In the culturing the transformant, the culturing conditions can be appropriately set depending on a type of the host used for producing the transformant. The culture may be a culturing supernatant, a transformant such as a cultured cell or a cultured bacterial cell, or a processed product or a disrupted product of these cultures.

When the prorenin receptor peptide is produced in a host after the culturing, the production method of the present disclosure includes, for example, isolating the prorenin receptor peptide by disrupting the host. When the prorenin receptor peptide is produced or secreted outside the host, the production method of the present disclosure uses a culture solution as it is, or removes the host by centrifugation or the like, for example. Thereafter, the production method of the present disclosure can isolate or purify the prorenin receptor peptide by using, for example, a general biochemical method used for isolating and purifying proteins. Specifically, condensation by an ultrafiltration membrane; salting out by a method such as ammonium sulfate precipitation; or chromatography using various columns such as gel filtration, ion exchange chromatography, and affinity chromatography, may be used alone or in combination as appropriate.

In the production method of the present disclosure, the prorenin receptor peptide may be obtained by *in vitro* translation using a cell-free synthesis system. In this case, the production method of the present disclosure may be performed by a method using RNA encoding the prorenin receptor peptide as a template, or a method using DNA encoding the prorenin receptor peptide as a template (via transcription and translation). As the cell-free synthesis system, a commercially available system, specifically for example, an EXPRESSWAY^{™} (trademark) system (manufactured by Invitrogen Corporation) or the like can be used. After the translation, the production method of the present disclosure can isolate or purify the prorenin receptor peptide by using, for example, a general biochemical method used for isolating and purifying proteins. Specifically, condensation by an ultrafiltration membrane; salting out by a method such as ammonium sulfate precipitation; or chromatography using various columns such as gel filtration, ion exchange chromatography, and affinity chromatography, may be used alone or in combination as appropriate. The isolation or purification can be performed, for example, in the same manner as in the case of using the transformant.

The prorenin receptor peptide obtained by the production method of the present disclosure may be used, for example, as a roughly purified product as it is, as a partially purified product, or as a purified single product.

In the production method of the present disclosure, the obtained prorenin receptor peptide may be pulverized by, for example, lyophilization, vacuum drying, spray drying, or the like. In this case, in the production method of the present disclosure, for example, the prorenin receptor peptide may be dissolved in a buffer such as an acetic acid buffer, a phosphoric acid buffer, a triethanolamine buffer, a tris hydrochloric acid buffer, and a GOOD buffer (e.g., PIPES, MES, and MOPS), in advance.

In the production method of the present disclosure, the obtained PRR peptide may further be bonded to the carrier protein. The binding may be determined based on a substituent (reactive group) at the binding position, depending on the binding position of the PRR peptide and the carrier protein.

### <Pharmaceutical Composition>

The present disclosures provide a pharmaceutical composition capable of inducing antibodies against PRR. The pharmaceutical composition of the present disclosure is a pharmaceutical composition including the prorenin receptor peptide, the conjugate, the nucleic acid, and/or the expression vector of the present disclosure and a pharmaceutically acceptable carrier. The pharmaceutical composition of the present disclosure is characterized by including the prorenin receptor peptide, the conjugate, the nucleic acid, and/or the expression vector (all of these are together hereinafter also referred to as the "active component") as an active component, and other configurations and conditions are not particularly limited. The pharmaceutical composition of the present disclosure includes the prorenin receptor peptide or the conjugate, or the nucleic acid encoding the prorenin receptor peptide or the conjugate, as an active component, and thus can exhibit an antitumor effect and/or an antimuscle-reduction effect when being used as an active component of a vaccine. The pharmaceutical composition of the present disclosure may incorporate the description of the conjugate.

The pharmaceutical composition of the present disclosure is capable of inducing antibodies against the prorenin receptor peptide or the conjugate, or the nucleic acid encoding these, for example, by being administered to an administration subject. Thus, the pharmaceutical composition of the present disclosure can also be referred to as a vaccine, a vaccine composition, or a vaccine formulation.

The pharmaceutical composition of the present disclosure includes a pharmaceutically acceptable carrier. The carrier may be, for example, a suspension, a solubilizer, a stabilizer, an isotonicifier, a preservative, an adsorption preventing agent, a surfactant, a diluent, a media, a pH adjuster, a soothing agent, a buffer, a sulfur-containing reducing agent, an antioxidant, or the like for administering the active components, and can be added to the extent that they do not disturb the effect of the present disclosure.

The pharmaceutical composition of the present invention may further include, for example, an immunostimulant. Examples of the immunostimulant include aluminum hydroxide, aluminum phosphate, aluminum chloride, Freund's complete adjuvant, Freund's incomplete adjuvant, CpG oligonucleotide, Poly I:C, a Toll-like receptor stimulant such as lipopolysaccharide (LPS), cytokine, lymphokine, and chemokines. Examples of the cytokine and the lymphokine include interferon such as IFN-γ; proinflammatory cytokine such as TNF-α; interleukin such as IL-1, IL-2, IL-3, IL-4, IL-12, and IL-13; a growth factor such as granulocyte-macrophage (GM) colony-stimulating factor (GM-CSF), and granulocyte colony stimulating factor (G-CSF); Flt3 ligands; B7-1; and B7-2.

### <Method for Treating Tumor>

In another embodiment, the present disclosure provides a method for treating a tumor. The method of treating a tumor according to the present disclosure uses on a subject, the prorenin receptor peptide, the conjugate, the nucleic acid, the expression vector, and/or the pharmaceutical composition of the present disclosure (all of these are together hereinafter also referred to as the "active component").

The method for treating a tumor according to the present disclosure includes, for example, administering the active component to the subject. Administration conditions in the administering the active component may incorporate the description of the administration conditions of the conjugate of the present disclosure. The administration may be *in vitro* or *in vivo.*

The method for treating a tumor according to the present disclosure includes as the active component the prorenin receptor peptide, the conjugate, or the nucleic acid encoding these, and thus can induce production of antibodies against the prorenin receptor when being applied to the subject. Accordingly, the method for treating a tumor according to the present disclosure can inhibit a tumor.

### <Method for Treating Sarcopenia or Frail>

In another embodiment, the present disclosure provides a method for treating sarcopenia or frail. The treatment method according to the present disclosure is a method for treating a subject by using the prorenin receptor peptide, the conjugate, the nucleic acid, the expression vector, and/or the pharmaceutical composition of the present disclosure (all of these are together hereinafter also referred to as the "active component") for the subject. The treatment method of the present disclosure is characterized by using the prorenin receptor peptide, the conjugate, the nucleic acid, and/or the expression vector as the active component, and other processes and conditions are not particularly limited.

The method for treating sarcopenia or frail according to the present disclosure includes, for example, administering the active component to the subject. Administration conditions in the administering the active component may incorporate the description of the administration conditions in the conjugate of the present disclosure. The administration may be *in vitro* or *in vivo.*

The method for treating sarcopenia or frail according to the present disclosure includes the prorenin receptor peptide, the conjugate, or the nucleic acid encoding these as the active component, and thus can induce production of antibodies against the prorenin receptor when being applied to the subject. Accordingly, the method for treating sarcopenia or frail according to the present disclosure can inhibit sarcopenia or frail.

### <Use of Pharmaceutical Composition>

Disclosed in the present disclosure are the prorenin receptor peptide, the conjugate, the nucleic acid, the expression vector, and/or the pharmaceutical composition, or the use of these, for the treatment of tumors. Disclosed in the present disclosure are the prorenin receptor peptide, the conjugate, the nucleic acid, the expression vector, and/or the pharmaceutical composition, or the use of these, for the production of pharmaceuticals for treating tumors. Disclosed in the present disclosure are the prorenin receptor peptide, the conjugate, the nucleic acid, the expression vector, and/or the pharmaceutical composition, or the use of these, for the treatment of sarcopenia or frail. Disclosed in the present disclosure are the prorenin receptor peptide, the conjugate, the nucleic acid, the expression vector, and/or the pharmaceutical composition, or the use of these, for the production of pharmaceuticals for treating sarcopenia or frail.

### Examples

Hereinafter, the present invention will be described in detail with reference to Examples, but the present disclosure is not limited to the embodiments described in Examples.

### [Example 1]

It was verified that the PRR peptide of the present disclosure has inducibility of antibodies against PRR, and an anti-tumor effect.

### (1) Preparation of Prorenin Receptor Peptide Antigen

As peptide fragments of the human prorenin receptor protein (SEQ ID NO: 5), the 198-211th PRR1 peptide (PRR1, SEQ ID NO: 1), the 223-236th PRR2 peptide (PRR2, SEQ ID NO: 2), the 221-230th PRR3 peptide (PRR3, SEQ ID NO: 3), and the 224-233rd PRR4 peptide (PRR4, SEQ ID NO: 7) were prepared. The 200-213rd PRR0 peptide (PRR0, SEQ ID NO: 8) was prepared as a comparative example. Keyhole Limpet Hemocyanin (KLH) was linked to an N-terminus of an amino acid of each of the polypeptides as a carrier protein by a known method, to obtain PRR0-Cg, PRR1-Cg, PRR2-Cg, PRR3-Cg, and PRR4-Cg corresponding to the polypeptides 0-4. The each of the antigens was adjusted to 1mg/ml. A cross-linking agent (EMCS) was used in the linking to prepare a conjugate by crosslinking an amino group of the peptide with a thiol group of a side chain of a cysteine residue of the KLH.

PRR4 peptide (SEQ ID NO: 7)
   KRYGEDSEQ
PRR0 peptide (SEQ ID NO: 8)
   HKHLAKDHSPDLYS

### (2) Measurement of Antibody Titer and Examination of Antitumor Effect in PRR0

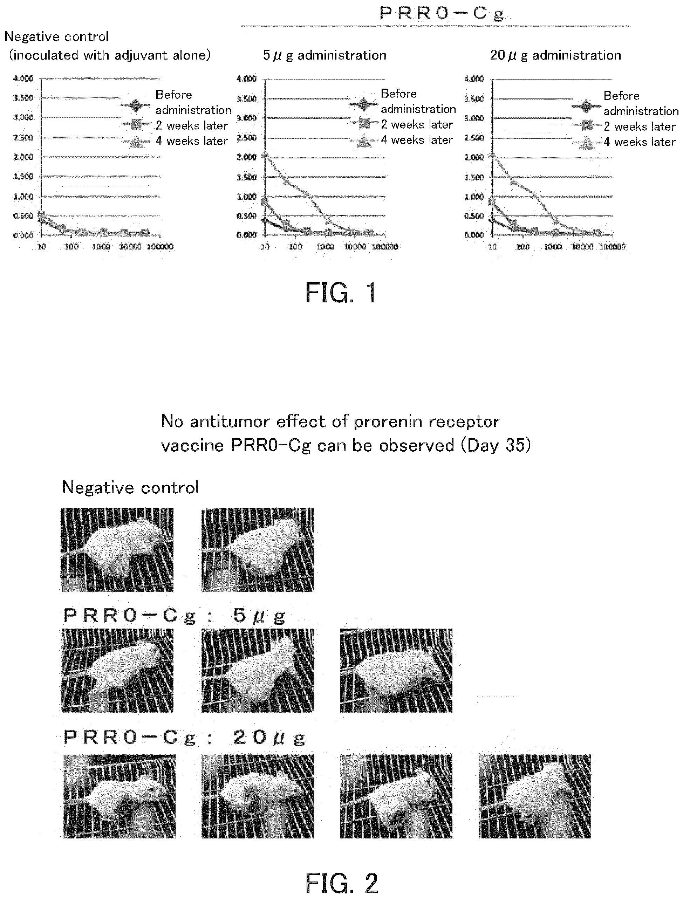

An antibody against PRR0 exhibits an antitumor effect *in vivo.* Thus, the PRR0-Cg as a prorenin receptor vaccine was inoculated into mice (BALB/c mice, 7 weeks old) for the measurement of the antibody titer against PRR (hereinafter referred to as the "antibody titer"), and the examination of the antitumor effect. Specifically, 5µg or 20µg of PRR0-Cg obtained in the Example 1(1) (1mg/ml) was inoculated into the mice together with an adjuvant (Freund's Complete Adjuvant, Cat No. 014-09541, manufactured by FUJIFILM Wako Pure Chemical Corporation) (n=4 for each). Serums were collected 2 and 4 weeks after the inoculation. 5 weeks after the first inoculation, 1 × 10⁶ cells of a mouse colorectal cancer cell CT26 were transplanted into each of the mice. The antibody titer in the serums was measured at the absorbance of 450nm. The effect on tumors was examined 10 weeks after the first inoculation. Measurement and examination for the negative control were performed in the same manner except that the adjuvant was inoculated alone into the mouse (n=2). These results are shown in FIGs. 1 and 2.

FIG. 1 shows graphs of the antibody titer of the prorenin receptor vaccine PRR0. In FIG. 1, the horizontal axis indicates the dilution ratio of the serum, and the vertical axis indicates the absorbance. As shown in FIG. 1, in the negative control mouse 2 weeks and 4 weeks after the first administration, no increase in the antibody titer was observed. Compared to the negative control, the antibody titer in the mice inoculated with PRR0-Cg tended to increase at 2 weeks and 4 weeks after the first administration, but exhibited no significant increase.

FIG. 2 shows images of mice 10 weeks after the first inoculation with the prorenin receptor vaccine PRR0-Cg (DAY 35: 5 weeks after transplantation of the mouse colorectal cancer cell CT26). In FIG. 2, the upper row shows the negative control mice, the middle row shows the mice inoculated with 5µg of PRR0-Cg, and the lower row shows the mice inoculated with 20µg of PRR0-Cg. As shown in FIG. 2, no antitumor effect was observed regardless of the dose of PRR0-Cg. These results show that PRR0 as the epitope does not function as a prorenin receptor vaccine, though the antibody against PRR0 exhibits the antitumor effect.

### (3) Measurement of Antibody Titer and Examination of Antitumor Effect of PRR1-4

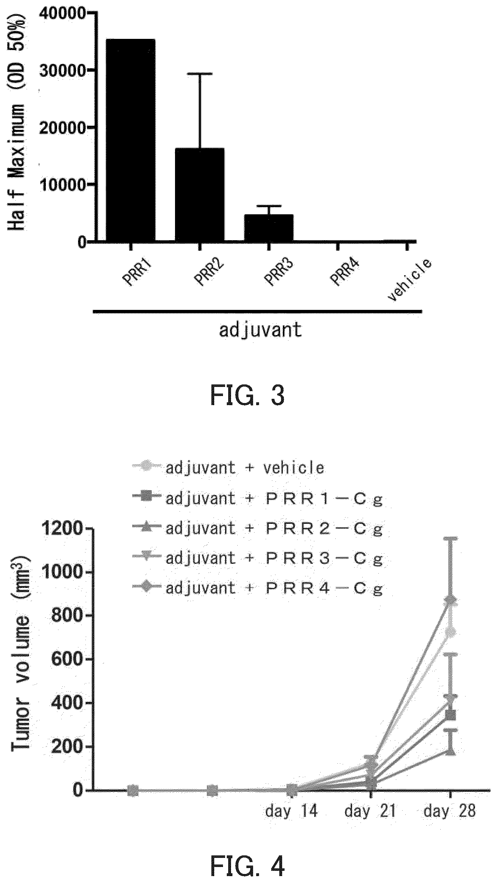

Next, the conjugate was inoculated into mice (BALB/c, 7 weeks old) as a prorenin receptor vaccine for the measurement of the antibody titer and the examination of the antitumor effect. Specifically, the mice were inoculated with 50µg of PRR1-Cg, PRR2-Cg, PRR3-Cg, or PRR4-Cg obtained in Example 1(1) and prepared at 1mg/ml, together with an adjuvant (Freund's Complete Adjuvant) (PRR1-Cg, 2-Cg, and 4-Cg: n=9, PRR3-Cg: n=8). 2 weeks after the first inoculation, the mice were inoculated again with 50µg of PRR1-Cg, PRR2-Cg, PRR3-Cg, or PRR4-Cg with the adjuvant (Freund's Incomplete Adjuvant). 3 weeks after the first inoculation, 1×10⁵ cells of a mouse colorectal cancer cell CT26 were transplanted into each of the mice. 7 weeks after the first inoculation, serums were collected from the mice. The antibody titer in the serums was measured as half value of the absorbance at 450nm (Half maximum). The tumor size of each of the mice was also measured 3, 4, 5, 6, and 7 weeks after the first inoculation. Measurement and examination for the negative control were performed in the same manner except that the adjuvant was inoculated alone into the mouse (n=7). These results are shown in FIGs. 3 and 4.

FIG. 3 is a graph showing the antibody titer of prorenin receptor vaccines PRR1-4, 7 weeks after the first administration (5 weeks after the second administration). In FIG. 3, the horizontal axis indicates the types of immunogens, and the vertical axis indicates half value of the absorbance at 450nm. As shown in FIG. 3, the antibody titer was found to be increased in the mice inoculated with PRR1-Cg, PRR2-Cg, and PRR3-Cg. Furthermore, the antibody titer was found to be significantly increased in the mice inoculated with PRR1-Cg and PRR2-Cg. On the other hand, no increase in the antibody titer was observed in the mouse inoculated with PRR4-Cg. These results show that the PRR1 peptide, the PRR2 peptide, and the PRR3 peptide of the present disclosure have the inducing function of antibodies against PRR.

FIG. 4 is a graph showing the antitumor effect of the prorenin receptor vaccines PRR1-4. In FIG. 4, the horizontal axis indicates the number of days lapsed after the transplantation of CT26, and the vertical axis indicates the tumor size (mm³). As shown in FIG. 4, the mice inoculated with PRR1-Cg, PRR2-Cg, or PRR3-Cg were found to have smaller tumors than that of the mouse inoculated with the adjuvant alone. On the other hand, in the mouse inoculated with PRR4-Cg, there was no change in the tumor size compared to that of the mouse inoculated with the adjuvant alone. These results show that the PRR1 peptide, the PRR2 peptide, and the PRR3 peptide of the present disclosure function as a prorenin receptor vaccine against colorectal cancer. Furthermore, the PRR1 peptide, the PRR2 peptide, and the PRR3 peptide can induce antibodies against PRR. Thus, is presumed that the PRR1 peptide, the PRR2 peptide, and the PRR3 exhibit the antitumor effect via the antibodies induced by these antigens.

### (4) Examination of Antitumor Effect of PRR2-3

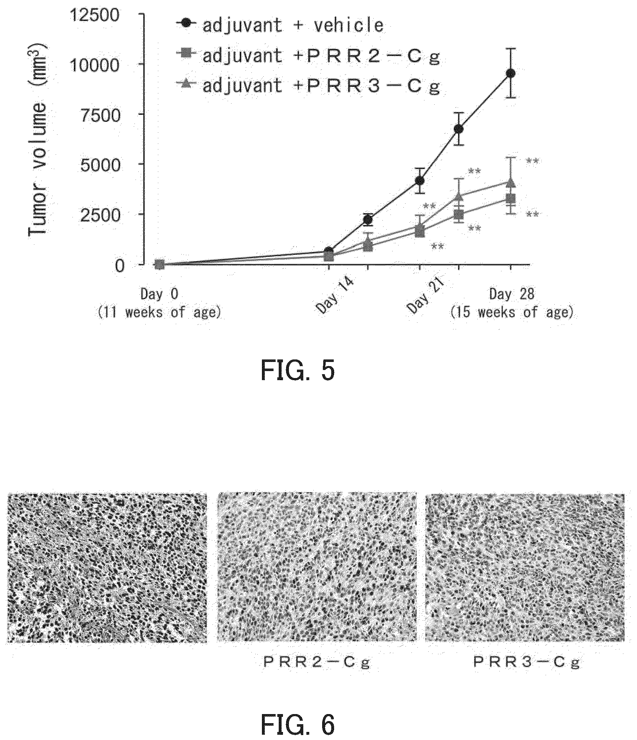

PRR2-Cg and PRR3-Cg which exhibited the antitumor effect were inoculated into mice (BALB/c, 6 weeks old) to reexamine the antitumor effect. Specifically, 50µg of PRR2-Cg or PRR3-Cg obtained in the Example 1(1) (1mg/ml) was inoculated into the mice, together with an adjuvant (Freund's Complete Adjuvant) (n=10 for each). Two weeks after the first inoculation, the mice were inoculated again with 50µg of PRR2-Cg or PRR3-Cg together with the adjuvant (Freund's Complete Adjuvant). 3 weeks after the first inoculation, 1× 10⁵ cells of a mouse colorectal cancer cell CT26 were transplanted subcutaneously into each of the mice. The tumor size in each of the mice was measured 14, 17, 21, 24, and 28 days after the transplantation of CT26 cells. Examination for the negative control was performed in the same manner except that the adjuvant was inoculated alone into the mouse (n=10). These results are shown in FIG. 5.

FIG. 5 is a graph showing the antitumor effect of the prorenin receptor vaccines PRR2 and PRR3. In FIG. 5, the horizontal axis indicates the number of days lapsed after the transplantation of CT26, and the vertical axis indicates the tumor size (mm³). As shown in FIG. 5, it was replicated that the tumor size in the mice inoculated with PRR2-Cg or PRR3-Cg was smaller than that of the mouse inoculated with the adjuvant alone. This result shows that the PRR2 peptide and PRR3 peptide of the present disclosure function as a prorenin receptor vaccine against colorectal cancer.

### (5) Examination of Antitumor Effect of PRR2-3 by Tissue Staining

β-catenin accumulation is found in the majority of colorectal cancers. Examination was performed by immunohistochemical staining to find whether the β-catenin accumulation in the large intestine is reduced by administration of PRR2-Cg or PRR3-Cg which exhibited the antitumor effect. Specifically, tumor tissue was collected from the mice administered with PRR2-Cg or PRR3-Cg obtained in the same manner as in the Example 1(3). Tumor tissue was also collected from a negative control in the same manner except that the mouse was inoculated with saline alone (n=5) instead of the peptide antigen. After the collection, the collected colon tissue was fixed using 4% paraformaldehyde. After the fixation, the fixed colon tissue was paraffin-embedded to prepare paraffin-embedded sections of 2µm thickness. The obtained paraffin-embedded sections were subjected to immunohistochemistry for β-catenin. As the primary antibody, an anti-β-catenin antibody (diluted 300 fold, Cat. No: 05-665, manufactured by Merck Millipore) was used for staining of β-catenin. A rabbit serum was used as a negative control. These results are shown in FIG. 6.

FIG. 6 shows images of the staining of activated β-catenin in colon tissue. In FIG. 6, the upper image shows stained colon tissue of the negative control, the left of the lower image shows stained colon tissue of the PRR2-Cg-inoculated mouse, and the right of lower image shows stained colon tissue of the PRR3-Cg-inoculated mouse. As shown in FIG. 6, little expression of activated β-catenin was observed in the colon tissue of the mice administered with PRR2-Cg or PRR3-Cg. These results show that the administration of the PRR2 peptide or the PRR3 peptide of the present disclosure allows inducing antibodies against PRR, thereby inhibiting the activation of the Wnt/β-catenin pathway.

### (6) Measurement of Antibody Titer of PRR2 and PRR3 in Short-term Observation

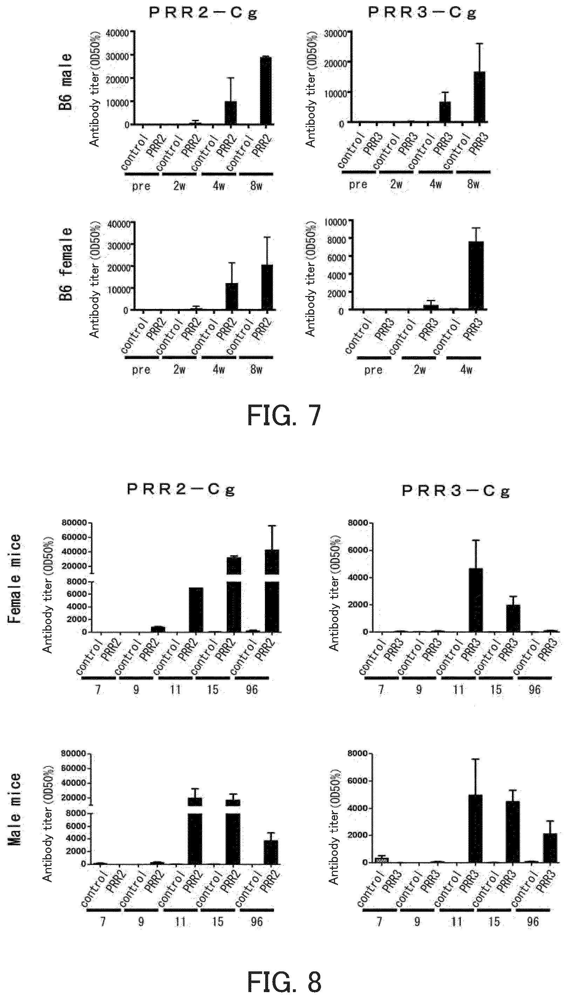

The antibody titer of PRR2-Cg and PRR3-Cg was measured for each sex of mice in short-term observations. Specifically, 50µg of PRR2-Cg or PRR3-Cg obtained in the Example 1(1) was inoculated into mice (C57BL/6 mice) together with an adjuvant (Freund's Complete Adjuvant), and serums were collected (n=10 for each). Two weeks after the first inoculation, the mice were inoculated again with 50µg of PRR2-Cg or PRR3-Cg together with the adjuvant (Freund's Incomplete Adjuvant). Serums were collected from the mice 2, 4, and 8 weeks after the first inoculation. The antibody titer in the serums was measured as half value of the absorbance of 450nm (Half maximum). Measurement for negative controls was performed in the same manner except that the adjuvant was inoculated alone in each of the mice. These results are shown in FIG. 7.

FIG. 7 shows graphs of the antibody titer of the prorenin receptor vaccines PRR2 and PRR3 in short-term observations. In FIG. 7, the upper graphs show the results of male mice, and the lower graphs show the results of female mice. In FIG. 7, the horizontal axis indicates the types of immunogens and the number of weeks lapsed after the first inoculation, and the vertical axis indicates half value of the absorbance of 450nm. As shown in FIG. 7, in the mice inoculated with PRR2-Cg or PRR3-Cg, the antibody titer was found to be increased 4 weeks after the first inoculation regardless of sex. These results suggest that the PRR2 peptide and PRR3 peptide can induce antibodies against PRR and function as a prorenin receptor vaccine regardless of sex.

### (7) Measurement of Antibody Titer of PRR2-3 in Long-term Observation

The antibody titer of PRR2-Cg and PRR3-Cg was measured for each sex of mice in long-term observations. Specifically, 50µg of PRR2-Cg or PRR3-Cg obtained in the Example 1(1) was inoculated into mice (C57BL/6 mice, 7 weeks old) together with an adjuvant (Freund's Complete Adjuvant), and serums were collected (n=10 for each). 2 weeks after the first inoculation, the mice were inoculated again with 50µg of PRR2-Cg or PRR3-Cg together with the adjuvant (Freund's Incomplete Adjuvant). Serums were collected from the mice 2, 4, 8 and 89 weeks after the first inoculation (corresponding to 9, 11, 15, and 96 weeks old). The antibody titer in the serums was measured as half value of the absorbance of 450nm (Half maximum). Measurement for negative controls was performed in the same manner except that the adjuvant was inoculated alone in each of the mice. These results are shown in FIG. 8.

FIG. 8 shows graphs of the antibody titer of the prorenin receptor vaccines PRR2 and PRR3 in long-term observations. In FIG. 8, the upper graphs show the results of male mice, and the lower graphs show the results of female mice. In FIG. 8, the horizontal axis indicates the types of immunogens and the weeks of age of the mice, and the vertical axis indicates half value of the absorbance of 450nm. As shown in FIG. 8, in the mice inoculated with PRR2-Cg, the antibody titer was found to be maintained 89 weeks after the first inoculation (96 weeks of age) regardless of sex. Further, in the male mice inoculated with PRR3-Cg, the antibody titer was found to be maintained 89 weeks after the first inoculation (96 weeks of age). These results show that the antibody titer of the PRR2 peptide is maintained in a long term regardless of sex. It was further suggested that the antibody titer of the PRR3 peptide is maintained in a long term, particularly in males.

### [Example 2]

It was verified that the PRR peptide of the present disclosure has inducibility of antibodies against the PRR and an antitumor effect, even when using CRM₁₉₇ as a carrier protein.

### (1) Examination of Carrier Protein in Mice

In the conjugates of the PRR2-Cg and the PRR3-Cg described above, KLH was used as a carrier protein. In the present example, instead of the KLH, a non-toxic mutant of diphtheria toxin CRM₁₉₇, which can be used in human clinics, was used. CRM₁₉₇ was linked to the N-terminus of the amino acid of the PRR2 polypeptide by a known method as a carrier protein to obtain CRM197-PRR2. The linking was performed in the same manner as in the linking of KLH, except for using CRM₁₉₇ instead of KLH.

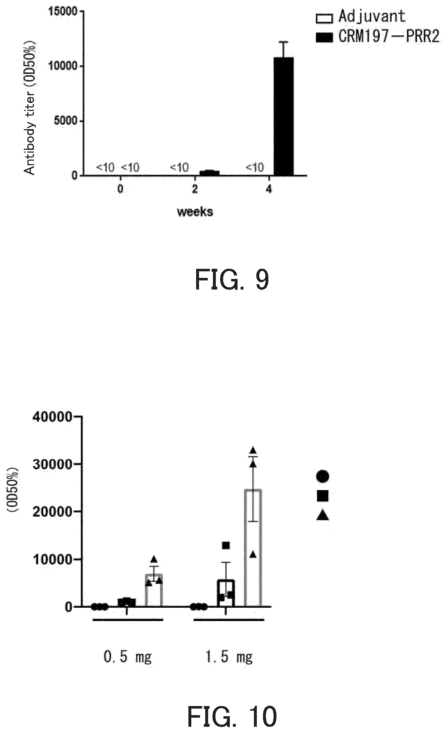

Next, examination was performed to find whether the CRM197-PRR2 functions as a prorenin receptor vaccine, similarly to KLH-conjugated PRR2. Specifically, the CRM197-PRR2 was inoculated into mice (C57BL/6 mice, 7 weeks old) to measure the antibody titer. The CRM197-PRR2 was prepared at the concentration of 7mg/ml, and further diluted by 10 times thereafter. After the dilution, the mice were inoculated with the dilutions containing 30µl of CRM197-PRR2 (containing approximately 20µg) together with 30µl of adjuvant (Freund's Complete Adjuvant) and serums were collected thereafter (n=10). 2 weeks after the first inoculation, the mice were inoculated again with 30µl of the CRM197-PRR2 together with the adjuvant (Freund's Incomplete Adjuvant). The serums of the mice were collected before the first inoculation, and 2 weeks and 4 weeks after the first inoculation. The antibody titer in the serums was measured as half value of the absorbance of 450nm (Half maximum). Examination for a negative control was performed in a similar manner except that mouse was inoculated with the adjuvant alone. These results are shown in FIG. 9.

FIG. 9 is a graph showing the antibody titer of CRM197-PRR2 in the mice. In FIG. 9, the horizontal axis indicates the number of weeks lapsed after the first administration, and the vertical axis indicates half value of the absorbance of 450nm. As shown in FIG. 9, the antibody titer was not increased in the mouse inoculated with the adjuvant alone. On the other hand, in the mice administered with CRM197-PRR2, there was a slight increase in the antibody titer 2 weeks after the first administration. In addition, in the mice administered with CRM197-PRR2, the antibody titer was found to be increased 4 weeks after the first administration. These results show that CRM197-conjugated PRR2 increases the antibody titer in mice, similarly to KLH-conjugated PRR2.

### (2) Examination of Carrier Protein in Monkey

It was verified that CRM197-PRR2 is efficacious in increasing the antibody titer in mice. Hence, examination was performed to find whether CRM197-PRR2 induces antibodies against PRR also in monkeys. Specifically, the CRM197-PRR2 was inoculated into cynomolgus monkeys to measure the antibody titer. The CRM197-PRR2 was prepared at the concentration of 7mg/ml. After the preparation, an adjuvant (2% aluminum hydroxide) of an amount equivalent to the CRM197-PRR2 was added to obtain a preparation at the concentration of 3.5mg/ml. Then, on days 1 and 29, the preparation was administered subcutaneously to the back (lumbar back) of the cynomolgus monkeys. The administration was performed at 140µl in the low dose group and 420µl in the high dose group. Serums of the cynomolgus monkeys were collected before the administration, and on days 29 and 57 after the administration. The antibody titer in the serums was measured as half value of the absorbance of 450nm. These results are shown in FIG. 10.

FIG. 10 is a graph showing the antibody titer of CRM197-PRR2 in cynomolgus monkeys. In FIG. 10, the horizontal axis indicates the inoculation groups and the number of days lapsed after the first administration, and the vertical axis indicates half value of the absorbance of 450nm. As shown in FIG. 10, the antibody titer was found to be increased with the lapse of days, in the low dose group and the high dose group. Further, the antibody titer was found to be increased dependently to the dose of CRM197-PRR2. These results show that CRM197-conjugated PRR2 increases the antibody titer in cynomolgus monkeys.

### [Example 3]

The β-catenin inhibitory effect, the antitumor effect, and the adverse reaction of the PRR peptide of the present disclosure were examined.

### (1) Examination of β-catenin Inhibition Effect of PRR2 and PRR3

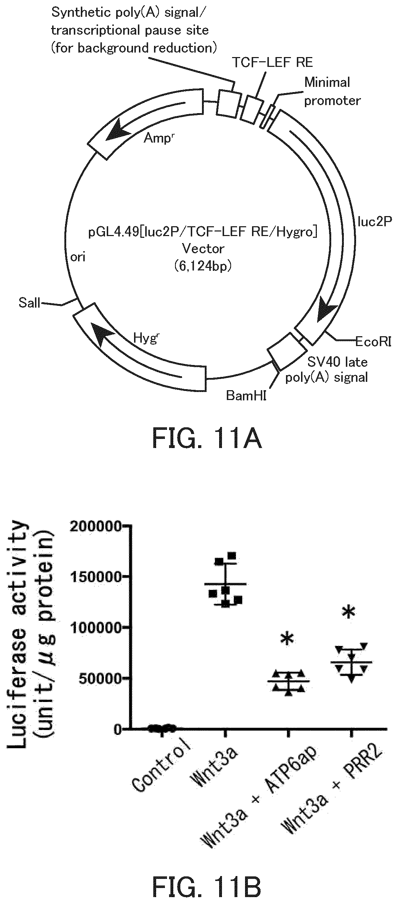

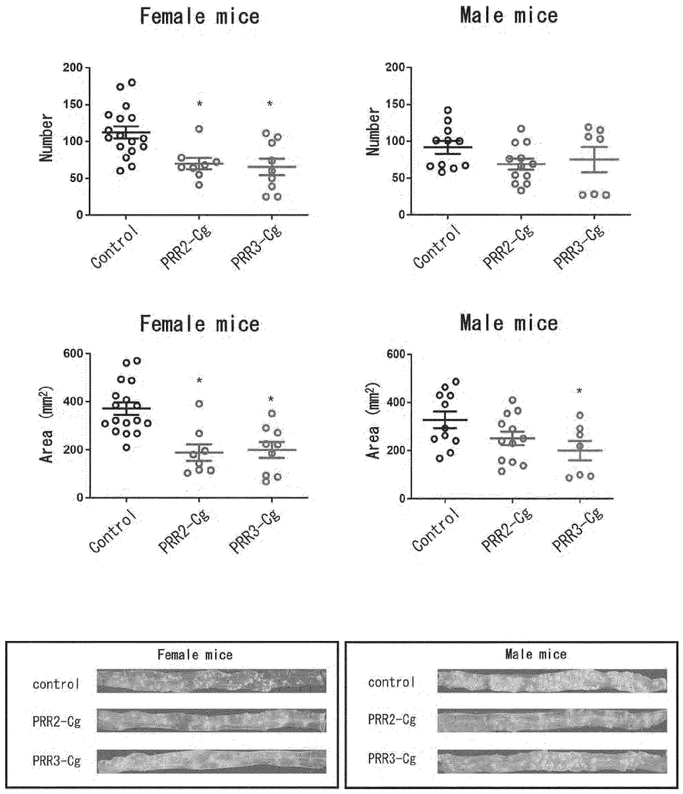

A luciferase reporter assay was used to examine whether PRR2-Cg with the antitumor effect reduces the activity of β-catenin which is activated by Wnt3a. Specifically, a pGL4.49 [luc2P TCF-LEF RE Hygro] vector (see FIG. 11A, manufactured by Promega Corporation) was made to be expressed in a cultured cells of HEK293 cell to construct a system (reporter cell) capable of evaluating the activation of β-catenin *in vitro.* In the reporter cell, activation of a Wnt/β-catenin pathway causes induction of expression of luciferase dependently to activated β-catenin. Therefore, the activity of Wnt/β-catenin pathway can be measured by measuring the luciferase activity of the reporter cell. Wnt3a was added to HEK293 cell, simultaneously with a ATP6ap monoclonal antibody (Clone67_3, see WO 2018/084236 A1) or a PRR2 purified antibody, and the HEK293 cell was cultured at 37°C for 24 hours. After the culturing, 50µl of Luciferase assay system (manufactured by Promega Corporation) containing a substrate of luciferase was added, and the luminescence was measured using Centro XS3 LB 960 (manufactured by Berthold Technologies GmbH & Co.KG). Measurement for the control group was performed in the same manner except that Wnt3a and each of the antibodies were not added. These results are shown in FIG. 11.

FIG. 11B is a graph showing the activity of β-catenin in HEK293 cells. In FIG. 11B, the horizontal axis indicates the addition groups, and the vertical axis indicates the luciferase activity. As shown in FIG. 11B, the activity of β-catenin was found to be inhibited by the ATP6ap monoclonal antibody and the PRR2 purified antibody. These results show that the PRR2 of the present disclosure can induce antibodies capable of inhibiting the Wnt/β-catenin pathway. It was also suggested that the PRR2 of the present disclosure can inhibit cancer by inducing antibodies capable of inhibiting the Wnt/β-catenin pathway.

### (2) Inhibitory Effect of PRR2 and PRR3 on Polyp Formation in APC^{min/+} Mice

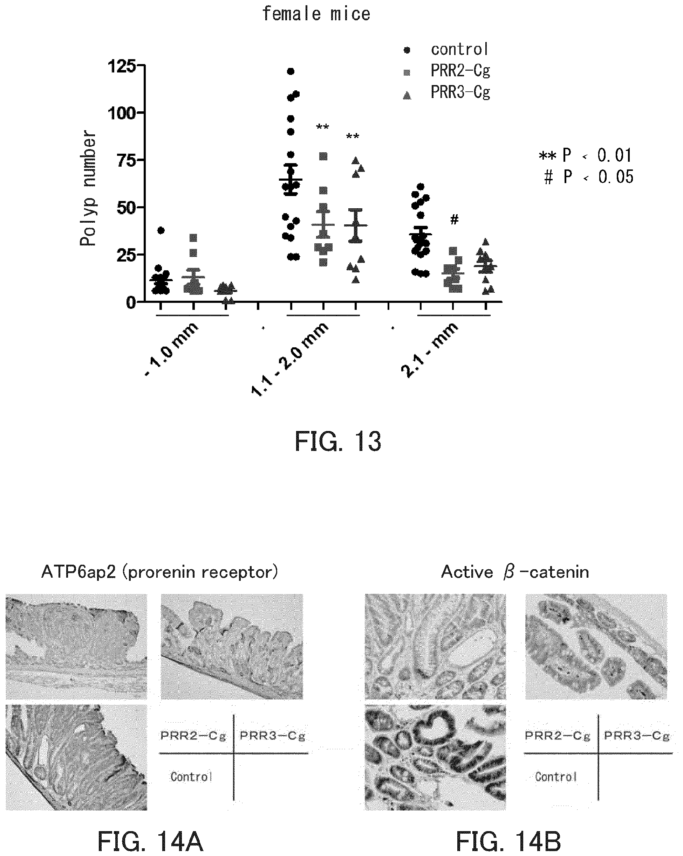

Examination was performed to find whether PRR2-Cg and PRR3-Cg, which were shown to have the antitumor effect, can inhibit polyp formation in colorectal cancer (adenomatosis polyposis) model mice. Specifically, under the same test protocol as in the Example 1(3) above, the adenomatosis polyposis model mice (C57BL/6J-Apcmin/+) were administered with saline, PRR2-Cg, or PRR3-Cg, together with an adjuvant. The intestines (large intestines and small intestines) were collected from the mice at 16-20 weeks of age. After the collection, intestinal polyps were analyzed. These results are shown in FIGs. 12A, 12B and 13.

FIGs. 12A and 12B show graphs of the inhibitory effect on polyp formation in the adenomatosis polyposis model mice. FIG. 12A shows a graph with respect to the total polyp numbers, FIG. 12B shows a graph with respect to the total polyp area (mm²), and FIG. 12C shows images in the cases of inoculating the adjuvant alone, PRR2-Cg, or PRR3-Cg. In FIG. 12A, the horizontal axis indicates the inoculation groups, and the vertical axis indicates the number of polyps. In FIG. 12B, the horizontal axis indicates the inoculation groups, and the vertical axis indicates the area value (mm²). As shown in FIGs. 12A and 12B, compared to the group inoculated with the adjuvant alone, the total polyp number and the total polyp area were found to be reduced in the groups inoculated with PRR2-Cg or PRR3-Cg. These results show that the PRR2 peptide and the PRR3 peptide of the present disclosure are effective in inhibiting polyp formation.

FIG. 13 is a graph showing the inhibitory effect on polyp formation in the adenomatosis polyposis model mice. In FIG. 13, the horizontal axis indicates the size (mm²) of the polyp, and the vertical axis indicates the number of polyps. As shown in FIG. 13, compared to the adenomatosis polyposis model mice inoculated with saline (Control), the inoculation with PRR2-Cg or PRR3-Cg was found to reduce the numbers of medium-sized (1.1-2.0mm²) and large-sized (2.1mm² or larger) polyps. These results suggest that the PRR2 peptide and the PRR3 peptide of the present disclosure suppresses the growth of polyps by inhibiting ATP6ap2.

### (3) Examination of the suppression of the β-catenin activity by PRR2 or PRR3 in APC^{min/+} Mice by Immunostaining

Immunostaining was used to examine whether the activity of β-catenin was inhibited by PRR2 and PRR3, which showed the inhibitory effect on polyp formation in the adenomatosis polyposis model mice. Specifically, under the same test protocol as in the Example 1(3) above, the adenomatosis polyposis model mice (C57BL/6J-Apcmin/+) were administered with saline, PRR2-Cg, or PRR3-Cg, together with an adjuvant. The intestines were collected from the mice at 16-20 weeks of age. After the collection, the collected intestines were fixed using 4% paraformaldehyde. After the fixation, the fixed intestines were paraffin-embedded to prepare paraffin-embedded sections of 2µm thickness. The obtained paraffin-embedded sections were subjected to immunostaining for β-catenin. As the primary antibody, an anti-ATP6ap2 antibody (diluted by 3000 fold, provided by Tohoku Pharmaceutical University: See Takuo Hirose et al., "Gene expression of (pro) renin receptor is upregulated in hearts and kidneys of rats with congestive heart failure," Peptides, Volume 30, Issue 12, 2009, Pages 2316-2322) was used for the staining of ATP6ap2 (prorenin receptor: PRR), and an anti-active β-catenin antibody (diluted by 1000 fold, Cat. No: 05-665, manufactured by Merck Millipore) was used for the staining of active β-catenin. As the secondary antibody, horseradish peroxidase-conjugated anti-rabbit IgG (manufactured by NICHIREI BIOSCIENCES INC.) was used. These results are shown in FIGs. 14A and 14B.

FIGs. 14A and 14B show images of the staining of ATP6ap2 or active β-catenin in the intestine. FIG. 14A shows the image of stained ATP6ap2, and FIG. 14B shows the image of stained active β-catenin. As shown in FIG. 14A, the staining of ATP6ap2 was strongly observed in intestinal polyps of the mice inoculated with saline. On the other hand, the staining of ATP6ap2 was attenuated in intestinal polyps of the mice inoculated with PRR2-Cg or PRR3-Cg. As shown in FIG. 14B, the staining of active β-catenin was strongly observed in the nucleus of the intestine of the mice inoculated with saline (Control). On the other hand, the staining of the active β-catenin was attenuated in the nucleus of the intestines of the mice inoculated with PRR2-Cg or PRR3-Cg. These results show that the PRR2 peptide and the PRR3 peptide of the present disclosure inhibit the expression of ATP6ap2 and the activity of β-catenin.

### (4) Examination of Adverse reaction by PRR2 and PRR3

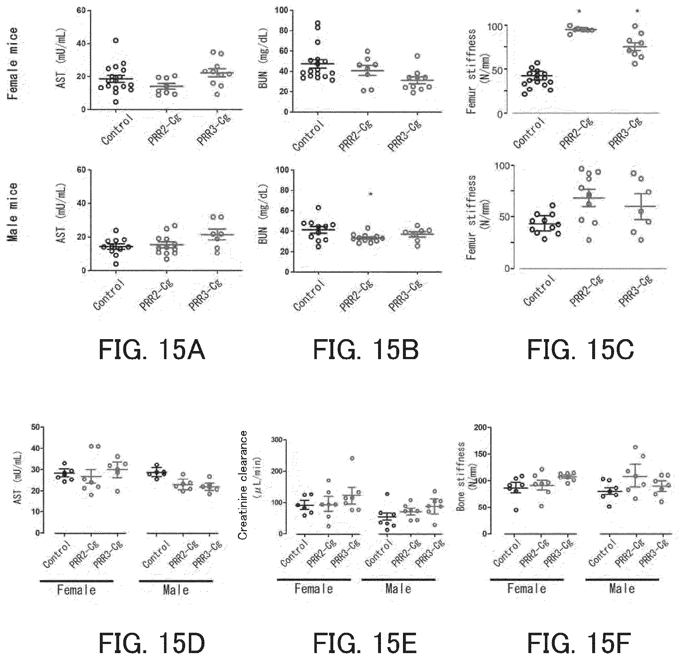

In Wnt/β-catenin signal inhibitors, adverse reactions such as bone lesion have been problematic. Thus, examination was performed to find whether the inoculation of PRR2 and PRR3 causes adverse reactions such as bone lesion. Specifically, under the same test protocol as in the Example 1(3) above, adenomatosis polyposis model mice (C57BL/6J-Apcmin/+) and normal mice (C57BL/6) were administered with saline, PRR2-Cg, or PRR3-Cg, together with an adjuvant. Adverse reactions were observed in the adenomatosis polyposis model mice 24 weeks after the administration. As indicators of adverse reactions in the adenomatosis polyposis model mice, AST (liver), BUN (kidney), and the bone strength were measured. Further, adverse reactions were observed in the normal mice 96 weeks after the administration. As indicators of adverse reactions in the normal mice, AST (liver), creatinine clearance (kidney), and the bone strength were measured. These results are shown in FIGs. 15A to 15F.

FIGs. 15A to 15F show graphs of the results of adverse reactions in the adenomatosis polyposis model mice and the normal mice. In FIG. 15A to 15C, the upper graphs show the results in female mice, and the lower graphs show the results in male mice. FIG. 15A shows AST (liver) in the adenomatosis polyposis model mice, FIG. 15B shows BUN (kidney) in the adenomatosis polyposis model mice, and FIG. 15C shows the bone strength in the adenomatosis polyposis model mice. FIG. 15D shows AST (liver) in the normal mice, FIG. 15E shows creatinine clearance (kidney) in the normal mice, and FIG. 15F shows the bone strength in the normal mice. In FIG. 15A, the horizontal axis indicates the inoculation groups, and the vertical axis indicates the AST enzymatic concentration. In FIG. 15B, the horizontal axis indicates the inoculation groups, and the vertical axis indicates the BUN blood concentration. In FIG. 15C, the horizontal axis indicates the inoculation groups and sex, and the vertical axis indicates the bone strength. In FIG. 15D, the horizontal axis indicates the inoculation groups and sex, and the vertical axis indicates the AST enzymatic concentration. In FIG. 15E, the horizontal axis indicates the inoculation groups and sex, and the vertical axis indicates the creatinine clearance value. In FIG. 15F, the horizontal axis indicates the inoculation groups and sex, and the vertical axis indicates the bone strength. As shown in FIGs. 15A to 15C, the adenomatosis polyposis model mice inoculated with PRR2-Cg or PRR3-Cg showed the same AST enzymatic concentration as the adenomatosis polyposis model mice inoculated with saline (Control). The adenomatosis polyposis model mice inoculated with PRR2-Cg or PRR3-Cg also showed decrease in the BUN blood concentration and increase or increase tendency in the bone strength, compared to the adenomatosis polyposis model mice inoculated with saline. As shown in FIGs. 15D to 15F, the normal model mice inoculated with PRR2-Cg or PRR3-Cg showed the same AST enzymatic concentration, the BUN blood concentration, and the bone strength as the normal mice inoculated with saline. These results show that the PRR2 peptide and the PRR3 peptide of the present disclosure improve the decrease in the kidney function and the bone strength caused by the adenoma, without causing the adverse reaction.

### (4) Examination of Improvement in Survival Rate with PRR2 and PRR3

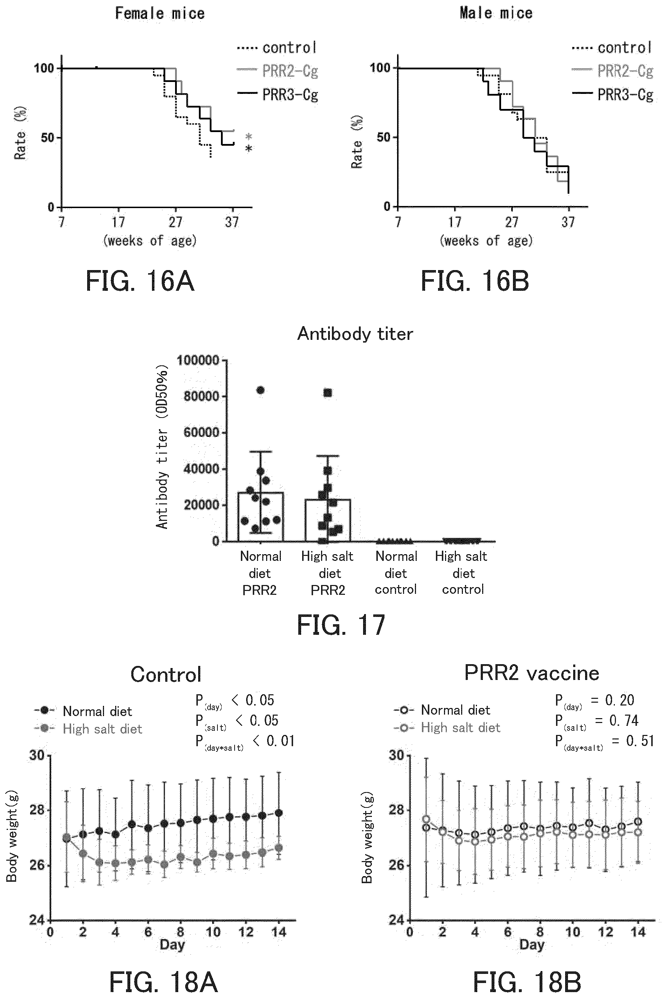

Many adenomatosis polyposis model mice die of intestinal obstruction, hemorrhage, and the like associated with adenoma increase. Thus, examination was performed to find whether the inoculation with PRR2-Cg and PRR3-Cg increases survival rate. Specifically, under the same test protocol as in Example 1(3) above, adenomatosis polyposis model mice (C57BL/6J-Apcmin/+) were administered with saline, PRR2-Cg, or PRR3-Cg, together with an adjuvant. The survival rates of the mice were measured during 30 weeks after the administration. When the body weight of the mouse decreased by 10%, the mouse was considered to be dead in the measurement of the survival rate. These results are shown in FIG. 16.

FIG. 16 shows graphs of the survival rate of the adenomatosis polyposis model mice. In FIG. 16, the horizontal axis indicates the number of weeks lapsed after the first inoculation, and the vertical axis indicates the survival rate. As shown in FIG. 16, the survival rate of the female mice inoculated with PRR2-Cg or PRR3-Cg was found to be increased compared to the survival rate of the mice inoculated with saline (Control). These results show that the PRR2 peptide and the PRR3 peptide of the present disclosure are useful for improving the prognosis.

### [Example 4]

It was verified that the PRR peptide of the present disclosure is effective in suppressing muscle-reduction.

Examination was performed to find whether the novel peptide of the present disclosure has a suppression effect on muscle mass reduction. High salt diet is known to cause muscle mass reduction in mice. Specifically, 20µg of CRM197-PRR2 prepared in the Example 2(1) was mixed with 30µl of saline and an adjuvant (Freund's Complete Adjuvant, Cat No. F5881, manufactured by Sigma-Aldrich) to prepare a PRR2 vaccine for the first administration. After the preparation, the PRR2 vaccine for the first administration was inoculated into mice (C57BL/6J mice, male, 8 weeks old). 2 weeks after the administration, 20µg of CRM197-PRR2-Cg prepared in the Example 2(1) was mixed with 30µl of saline and an adjuvant (Freund's Complete Adjuvant, Cat No. F5881, manufactured by Sigma-Aldrich) to prepare a PRR2 vaccine for the second administration. After the preparation, the PRR2 vaccine for the second administration was inoculated into the mice. 4 weeks after the second administration, high salt loading was initiated. The high saline loading was performed by feeding the mice with high salt diet for 2 weeks. Since the high salt loading causes the reduction in skeletal muscle mass in mice, the high salt loading would be a model of sarcopenia or frail. The experimental groups were 4 groups: (i) inoculated with the adjuvant alone + fed with normal diet (n=10), (ii) inoculated with the adjuvant alone + fed with high salt diet (n=10), (iii) inoculated with the PRR2 vaccine + fed with normal diet (n=10), and (iv) inoculated with the PRR2 vaccine + fed with high salt diet (n=10). The groups inoculated with the adjuvant alone were inoculated with saline instead of the PRR2 vaccine. Serums were collected from the mice 6 weeks after the first inoculation. The antibody titer in the serums was measured at the absorbance of 450nm. These results are shown in FIG. 17 and FIGs. 18A and 18B.

FIG. 17 is a graph showing the antibody titer of the PRR2 vaccine. In FIG. 17, the horizontal axis indicates the experimental groups, and the vertical axis indicates the half value of the absorbance of 450nm. As shown in FIG. 17, the antibody titer in the mice inoculated with PRR2-Cg was found to be increased regardless of the high salt loading. On the other hand, no increase in the antibody titer was observed in the mice of the control groups inoculated with the adjuvant, regardless of the high salt loading.

FIGs. 18A and 18B are graphs showing the body weight shift due to PRR2-Cg. FIG. 18A shows the weight shift of the control groups (i) and (ii) administered with the adjuvant alone, and FIG. 18B shows the weight shift of groups (iii) and (iv) inoculated with the PRR2 vaccine. In FIGs. 18A and 18B, the horizontal axis indicates the number of days lapsed after the initiation of the high salt loading, and the vertical axis indicates the body weight of the mice. As shown in FIGs. 18A and 18B, in the mice inoculated with the adjuvant alone, the body weight of the high salt diet group was found to be decreased compared to that of the normal diet group. On the other hand, in the mice inoculated with PRR2 vaccine, the body weight of the high salt diet group was similar to that of the normal diet group. These results show that the PRR2 peptide of the present disclosure is effective in suppressing muscle mass reduction.

While the present invention has been described above with reference to illustrative example embodiments, the present invention is by no means limited thereto. Various changes and variations that may become apparent to those skilled in the art may be made in the configuration and specifics of the present invention without departing from the scope of the present invention.

This application claims priority from Japanese Patent Application No. 2022-032709 filed on March 3, 2022. The entire subject matter of the Japanese Patent Application is incorporated herein by reference.

### Industrial Applicability

### <Supplementary Notes>

The whole or part of the example embodiments disclosed above can be described as, but not limited to, the following supplementary notes.

### <PRR Peptide>

### (Supplementary Note 1)

A prorenin receptor peptide, including a polypeptide of following (P1), (P2), or (P3):
(P1) a polypeptide consisting of an amino acid sequence of any one of SEQ ID NO: 1-3;
(P2) a polypeptide consisting of an amino acid sequence of any one of SEQ ID NO: 1-3 in which one or two or three amino acids are deleted, substituted, or added, or
(P3) a polynucleotide consisting of an amino acid sequence having 70% or more of identity to an amino acid sequence of any one of SEQ ID NO: 1-3.

### <Conjugate>

### (Supplementary Note 2)

A conjugate, including:
an antigenic peptide, and
a carrier protein, wherein
the antigenic peptide is bonded to the carrier protein, and
the antigenic peptide includes the prorenin receptor peptide according to Supplementary Note 1.

### (Supplementary Note 3)

The conjugate according to Supplementary Note 2, wherein
the carrier protein includes a cysteine residue, and
the antigenic peptide is bonded to the cysteine residue of the carrier protein.

### (Supplementary Note 4)

The conjugate according to Supplementary Note 2 or 3, wherein
the carrier protein is a diphtheria toxin or a mutant of the diphtheria toxin, or a tetanus toxin or a fragment of the tetanus toxin.

### (Supplementary Note 5)

The conjugate according to any one of Supplementary Notes 2 to 4, wherein
the carrier protein is CRM₁₉₇.

### (Supplementary Note 6)

The conjugate according to any one of Supplementary Notes 2 to 5, which induces formation of an antibody capable of inhibiting activity of a Wnt/β-catenin pathway in an activity assay of the Wnt/β-catenin pathway, when being administered to a subject.

### (Supplementary Note 7)

The conjugate according to any one of Supplementary Notes 2 to 6, for use in treatment of a tumor.

### (Supplementary Note 8)

The conjugate according to Supplementary Note 7, wherein
the tumor is prorenin receptor positive.

### (Supplementary Note 9)

The conjugate according to Supplementary Note 7 or 8, wherein
the tumor is familial adenomatous polyposis.

### (Supplementary Note 10)

The conjugate according to Supplementary Note 9, for use in treatment of familial adenomatous polyposis causing at least one complication selected from the group consisting of gastrointestinal benign multiple neoplasia, Gardner's syndrome, Turcot's syndrome, and Desmoid Fibromatosis.

### (Supplementary Note 11)

The conjugate according to any one of Supplementary Notes 2 to 6, for use in treatment of sarcopenia or frail.

### <Nucleic Acid>

### (Supplementary Note 12)

A nucleic acid encoding the peptide according to Supplementary Note 1.

### <Expression Vector>

### (Supplementary Note 13)

An expression vector, including the nucleic acid according to Supplementary Note 12.

### (Supplementary Note 14)

The expression vector according to Supplementary Note 13, wherein
the expression vector is a viral vector.

### <Pharmaceutical Composition>

### (Supplementary Note 15)

A pharmaceutical composition, including:
at least one component selected from the group consisting of the prorenin receptor peptide according to Supplementary Note 1, the conjugate according to any one of Supplementary Notes 2 to 11, the nucleic acid according to Supplementary Note 12, and the expression vector according to Supplementary Note 13 or 14, and
a pharmaceutically acceptable carrier.

### (Supplementary Note 16)

The pharmaceutical composition according to Supplementary Note 15, further including an immunostimulant.

### (Supplementary Note 17)

The pharmaceutical composition according to Supplementary Note 15 or 16, which induces formation of an antibody capable of inhibiting activity of a Wnt/β-catenin pathway in an activity assay of the Wnt/β-catenin pathway, when being administered to a subject.

### <Use for Tumor Treatment>

### (Supplementary Note 18)

The pharmaceutical composition according to any one of Supplementary Notes 15 to 17, for use in treatment of a tumor.

### (Supplementary Note 19)

The pharmaceutical composition according to Supplementary Note 18, wherein the tumor is prorenin receptor positive.

### (Supplementary Note 20)

The pharmaceutical composition according to Supplementary Note 18 or 19, wherein the tumor is familial adenomatous polyposis.

### (Supplementary Note 21)

The pharmaceutical composition according to Supplementary Note 20, for use in treatment of familial adenomatous polyposis causing at least one complication selected from the group consisting of gastrointestinal benign multiple neoplasia, Gardner's syndrome, Turcot's syndrome, and Desmoid Fibromatosis.

### <Use for Sarcopenia or Frail>

### (Supplementary Note 22)

The pharmaceutical composition according to any one of Supplementary Notes 15 to 17, for use in treatment of sarcopenia or frail.

### <Transformant>

### (Supplementary Note 23)

A transformant, including the prorenin receptor peptide according to Supplementary Note 1, the nucleic acid according to Supplementary Note 12, or the expression vector according to Supplementary Note 13 or 14.

### <Production Method>

### (Supplementary Note 24)

A method for producing a prorenin receptor peptide, including
expressing at least one component selected from the group consisting of the nucleic acid according to Supplementary Note 12, and the expression vector according to Supplementary Note 13 or 14.

### (Supplementary Note 25)

The production method according to Supplementary Note 24, wherein
the expressing the component includes:
culturing the transformant according to Supplementary Note 23, and
isolating the prorenin receptor peptide.

### <Method for Treating Tumor>

### (Supplementary Note 26)

A method for treating a tumor, including
administering to a subject at least one component selected from the group consisting of the prorenin receptor peptide according to Supplementary Note 1, the conjugate according to any one of Supplementary Notes 2 to 10, the nucleic acid according to Supplementary Note 12, the expression vector according to Supplementary Note 13 or 14, and the pharmaceutical composition according to any one of Supplementary Notes 15 to 21.

### <Method for Treating Sarcopenia or Frail>

### (Supplementary Note 27)

A method for treating sarcopenia or frail, including
administering to a subject at least one component selected from the group consisting of the prorenin receptor peptide according to Supplementary Note 1, the conjugate according to any one of Supplementary Notes 2 to 6 and 11, the nucleic acid according to Supplementary Note 12, the expression vector according to Supplementary Note 13 or 14, and the pharmaceutical composition according to any one of Supplementary Notes 15 to 17 and 22.

### <Use>

### (Supplementary Note 28)

At least one component selected from the group consisting of the prorenin receptor peptide according to Supplementary Note 1, the conjugate according to any one of Supplementary Notes 2 to 10, the nucleic acid according to Supplementary Note 12, the expression vector according to Supplementary Note 13 or 14, and the pharmaceutical composition according to any one of Supplementary Notes 15 to 21, for use in treatment of a tumor.

### (Supplementary Note 29)

At least one component selected from the group consisting of the prorenin receptor peptide according to Supplementary Note 1, the conjugate according to any one of Supplementary Notes 2 to 6 and 11, the nucleic acid according to Supplementary Note 12, the expression vector according to Supplementary Note 13 or 14, and the pharmaceutical composition according to any one of Supplementary Notes 15 to 17 and 22, for use in treatment of sarcopenia or frail.

### Industrial Applicability

As described above, the present disclosure allows inducing antibodies against prorenin receptors. Further, the present disclosure allows indirectly inhibiting activity of a Wnt/β-catenin pathway. Accordingly, the prorenin receptor peptide of the present disclosure can, for example, treat diseases due to abnormality in the Wnt/β-catenin pathway. Therefore, the present disclosure is extremely useful in the field of pharmaceuticals and the like.

## Claims

1. A prorenin receptor peptide, comprising a polypeptide of following (P1), (P2), or (P3):
(P1) a polypeptide consisting of an amino acid sequence of any one of SEQ ID NO: 1-3;
(P2) a polypeptide consisting of an amino acid sequence of any one of SEQ ID NO: 1-3 in which one or two or three amino acids are deleted, substituted, or added, or
(P3) a polynucleotide consisting of an amino acid sequence having 80% or more of identity to an amino acid sequence of any one of SEQ ID NO: 1-3.

2. A conjugate, comprising:
an antigenic peptide, and
a carrier protein, wherein
the antigenic peptide is bonded to the carrier protein, and
the antigenic peptide includes the prorenin receptor peptide according to Claim 1.

3. The conjugate according to Claim 2, wherein
the carrier protein includes a cysteine residue, and
the antigenic peptide is bonded to the cysteine residue of the carrier protein.

4. The conjugate according to Claim 2 or 3, wherein
the carrier protein is a diphtheria toxin or a mutant of the diphtheria toxin, or a tetanus toxin or a fragment of the tetanus toxin.

5. The conjugate according to any one of Claims 2 to 4, wherein
the carrier protein is CRM₁₉₇.

6. The conjugate according to any one of Claims 2 to 5, which induces formation of an antibody capable of inhibiting activity of a Wnt/β-catenin pathway in an activity assay of the Wnt/β-catenin pathway, when being administered to a subject.

7. The conjugate according to any one of Claims 2 to 6, for use in treatment of a tumor.

8. The conjugate according to Claim 7, wherein
the tumor is prorenin receptor positive.

9. The conjugate according to Claim 7 or 8, wherein
the tumor is familial adenomatous polyposis.

10. The conjugate according to Claim 9, for use in treatment of familial adenomatous polyposis causing at least one complication selected from the group consisting of gastrointestinal benign multiple neoplasia, Gardner's syndrome, Turcot's syndrome, and Desmoid Fibromatosis.

11. The conjugate according to any one of Claims 2 to 6, for use in treatment of sarcopenia or frail.

12. A nucleic acid encoding the peptide according to Claim 1.

13. An expression vector, comprising the nucleic acid according to Claim 12.

14. The expression vector according to Claim 13, wherein
the expression vector is a viral vector.

15. A pharmaceutical composition, comprising:
at least one component selected from the group consisting of the prorenin receptor peptide according to Claim 1, the conjugate according to any one of Claims 2 to 11, the nucleic acid according to Claim 12, and the expression vector according to Claim 13 or 14, and
a pharmaceutically acceptable carrier.

16. The pharmaceutical composition according to Claim 15, further comprising an immunostimulant.

17. The pharmaceutical composition according to Claim 15 or 16, which induces formation of an antibody capable of inhibiting activity of a Wnt/β-catenin pathway in an activity assay of the Wnt/β-catenin pathway, when being administered to a subject.

18. The pharmaceutical composition according to any one of Claims 15 to 17, for use in treatment of a tumor.

19. The pharmaceutical composition according to Claim 18, wherein
the tumor is prorenin receptor positive.

20. The pharmaceutical composition according to Claim 18 or 19, wherein
the tumor is familial adenomatous polyposis.

21. The pharmaceutical composition according to Claim 20, for use in treatment of familial adenomatous polyposis causing at least one complication selected from the group consisting of gastrointestinal benign multiple neoplasia, Gardner's syndrome, Turcot's syndrome, and Desmoid Fibromatosis.

22. The pharmaceutical composition according to any one of Claims 15 to 17, for use in treatment of sarcopenia or frail.

23. A transformant, comprising the prorenin receptor peptide according to Claim 1, the nucleic acid according to Claim 12, or the expression vector according to Claim 13 or 14.

24. A method for producing a prorenin receptor peptide, comprising
expressing at least one component selected from the group consisting of the nucleic acid according to Claim 12, and the expression vector according to Claim 13 or 14.

25. The production method according to Claim 24, wherein
the expressing the component includes:
culturing the transformant according to Claim 23, and
isolating the prorenin receptor peptide.

26. A method for treating a tumor, comprising
administering a subject with at least one component selected from the group consisting of the prorenin receptor peptide according to Claim 1, the conjugate according to any one of Claims 2 to 10, the nucleic acid according to Claim 12, the expression vector according to Claim 13 or 14, and the pharmaceutical composition according to any one of Claims 15 to 21.

27. A method for treating sarcopenia or frail, comprising
administering a subject with at least one component selected from the group consisting of the prorenin receptor peptide according to Claim 1, the conjugate according to any one of Claims 2 to 6 and 11, the nucleic acid according to Claim 12, the expression vector according to Claim 13 or 14, and the pharmaceutical composition according to any one of Claims 15 to 17 and 22.

28. At least one component selected from the group consisting of the prorenin receptor peptide according to Claim 1, the conjugate according to any one of Claims 2 to 10, the nucleic acid according to Claim 12, the expression vector according to Claim 13 or 14, and the pharmaceutical composition according to any one of Claims 15 to 21, for use in treatment of a tumor.

29. At least one component selected from the group consisting of the prorenin receptor peptide according to Claim 1, the conjugate according to any one of Claims 2 to 6 and 11, the nucleic acid according to Claim 12, the expression vector according to Claim 13 or 14, and the pharmaceutical composition according to any one of Claims 15 to 17 and 22, for use in treatment of sarcopenia or frail.
